# EUROPEAN PATENT SPECIFICATION

(11) **EP 3 612 111 B1**
(45) Date of publication and mention of the grant of the patent: **04.11.2026**
(21) Application number: 18721182.6
(22) Date of filing: 27.03.2018
(51) Int. Cl.: A61B 17/34, A61B 90/11

(54) **SURGICAL NAVIGATION SYSTEMS**
CHIRURGISCHE NAVIGATIONSSYSTEME
SYSTÈMES DE NAVIGATION CHIRURGICALE

(30) Priority: 21.04.2017 US 201762488192 P; 23.03.2018 US 201815934165
(43) Date of publication of application: 26.02.2020
(73) Proprietor: ClearPoint Neuro, Inc., Solana Beach, CA 92075 (US)
(72) Inventor: PANDEY, Rajesh, Irvine, California 92604 (US); DALY, Maxwell Jerad, Redlands, California 92373 (US); PIFERI, Peter G., Orange, California 92867 (US)
(74) Representative: Marks & Clerk LLP
(86) International application number: PCT/US2018/024515
(87) International publication number: WO 2018/194800

(56) References cited:
- WO-A1-2015/057807
- WO-A1-2015/057807
- WO-A2-2009/042130
- WO-A2-2009/042130
- US-A1- 2003 055 436
- US-A1- 2006 190 054
- US-A1- 2009 171 184
- US-A1- 2009 171 184

## Description

### FIELD OF THE INVENTION

The present invention relates generally to medical systems and, more particularly, to in vivo medical systems.

### BACKGROUND

Surgical navigation systems identify desired trajectories and paths to target tissue or anatomy during surgeries for introducing medical interventional devices into the body. *See*, U.S. Patent No. 9,042,958 and U.S. Patent No. 9,498,290. In particular, WO 2015/057807 (a corresponding application to US 9,498,290) describes image guided surgical systems. The systems have a trajectory frame which removably secures different components for providing a desired intrabody trajectory. The components may include one or more of an optical or EM tracking/navigation probe, and a user interface and circuit configured to provide a navigation system with a registration of physical surgical space to imaging space using an optical and/or EM navigation and related markers or fiducials.

### SUMMARY

In the disclosure described methods are disclosed for illustrative purposes only and have not been claimed. The invention is limited by the scope of independent claim 1 and further optional embodiments are disclosed in the dependent claims. Embodiments of the present invention provide devices and systems which can employ a system with a trajectory guide assembly that can serially and interchangeably hold either or both a fluid-filled single lumen guide or a fluid-filled multi-lumen guide and one or more elongated device guides for localized placement and/or delivery of diagnostic or therapeutic devices or substances.

Embodiments of the present invention may be particularly suitable for introducing therapeutic medications using an intrabody cannula, placing neuro-modulation leads, such as Deep Brain Stimulation ("DBS") leads, implantable parasympathetic or sympathetic nerve chain leads and/or CNS stimulation leads, as well as other devices within the brain.

Embodiments of the present invention may be suitable for a number of interventional procedures in many locations inside the body including, but not limited to, brain, cardiac, spinal, urethral, and the like.

Embodiments of the present invention may be suitable for a number of image guided drug delivery procedures to intra-brain or other intra-body targeted locations.

Embodiments of the present invention may be suitable for a number of image-guided tumor removal procedures.

Examples of the present disclosure are directed to surgical navigation systems that include a trajectory guide assembly with a base having a patient access aperture formed therein. The base is configured to be secured to the body of a patient; a yoke movably mounted to the base and rotatable about a first axis. The assembly also includes a platform with an open port that is movably mounted to the yoke and rotatable about a second axis. The system also includes a trajectory selection guide member comprising at least one longitudinally extending fluid filled channel of one or more contrast agents releasably attachable to the platform; and a multi-lumen device guide comprising a plurality of longitudinally extending open channels releasably attachable to the platform. The trajectory selection guide member and the multi-lumen device guide are serially interchangeably held by the platform and each have a length sufficient to extend through the port of the platform with a bottom portion thereof residing a distance below the platform.

The system can include an image processing circuit configured to generate and display a virtual trajectory selection guide member configured as a virtual multi-lumen guide array and aligned with an image of the trajectory guide assembly. The virtual multi-lumen guide array can include a plurality of radially and/or circumferentially spaced apart virtual channels spaced apart about a virtual center channel in a pattern corresponding to positions of the open channels of the multi-lumen device guide. The virtual center channel can be aligned with a center of the open port of the platform.

The platform can include visual orientation indicia on an upper surface thereof that includes a patient right directional indicator, a patient left directional indicator and a forward directional indicator.

The trajectory selection guide member can be a multi-lumen guide array with a plurality of radially and/or circumferentially spaced apart fluid filled lumens spaced apart about a center fluid filled lumen. The trajectory selection guide member can have an upper surface with visual orientation indicia including a patient right directional indicator, a patient left directional indicator and a forward directional indicator.

The trajectory selection guide member can have a cap sealably attached to and enclosing a primary body. The cap can reside above a liquid reservoir. The liquid reservoir can have a width that is larger than a width of the at least one longitudinally extending fluid filled lumen and merges into the at least one longitudinally extending fluid filled channel.

The platform can be rectangular. The system can also include a tubular support member held by the platform that extends under the open port. The open port of the platform can have a perimeter with an alignment feature that circumferentially extends about a sub-set of the perimeter and that slidably receives a matable alignment feature on the multi-lumen device guide.

The system further includes at least one drill bit guide that is also releasably and interchangeably extended through the port of the platform and is directly secured to the platform. The at least one drill bit guide is a rotatable offset guide with a longitudinally extending channel that is offset from an axially extending centerline of the guide and additional guides can have a center guide with a longitudinally extending channel that is centered with an axially extending centerline of the guide; and a rotatable combination guide with a center longitudinally extending channel that is aligned with an axially extending centerline of the guide and a radially offset longitudinally extending channel.

The trajectory selection guide member can be a multi-lumen guide array that comprises a plurality of radially and/or circumferentially spaced apart fluid filled lumens spaced apart about a center fluid filled lumen. The multi-lumen guide array and the multi-lumen device guide can have the same number of channels in the same array configuration.

The virtual multi-lumen guide array and the multi-lumen device guide can have the same number of channels in a common array configuration.

The trajectory selection guide member can be a multi-lumen guide array with a plurality of radially and/or circumferentially spaced apart fluid filled lumens. The fluid filled channels of the multi-lumen guide array terminate at a top end under a cap. The multi-lumen device guide can have a top end that is at the same height as the top end of the fluid filled channels.

The trajectory guide assembly can include a pair of arcuate laterally spaced apart arms that hold the platform therebetween and above the base and only two actuators for pitch and roll.

The trajectory guide assembly can be devoid of x-y direction actuators.

The platform can be slidably supported by the arms to thereby allow the mount to slidably travel forward and rearward over a curvilinear path defined by the arms.

The plurality of fluid filled channels can have a common length.

The trajectory selection guide member can be a multi-lumen guide array with a plurality of radially and/or circumferentially spaced apart fluid filled lumens spaced apart about a center fluid filled lumen.

The plurality of fluid filled channels of the multi-lumen guide array and the plurality of open channels of the device guide can be seven.

The trajectory selection guide member can be a multi-lumen guide array with a plurality of spaced apart longitudinally extending fluid filled lumens. The plurality of longitudinally extending fluid filled channels can include a center channel with adjacent channels residing spaced apart about the center channel. The multi-lumen guide array can include orientation indicia corresponding to patient directions of right, left and forward. The platform can have corresponding orientation indicia.

Yet other examples are directed to surgical navigation systems that include a trajectory guide assembly comprising: a base having a patient access aperture formed therein. The base is configured to be secured to the body of a patient. The assembly also includes a yoke movably mounted to the base and rotatable about an axis; and a platform with an open port that is movably mounted to the yoke and rotatable about an axis. The systems also include a trajectory selection guide comprising at least one longitudinally extending fluid filled channel of one or more contrast agents releasably attachable to the platform; and a multi-lumen device guide comprising a plurality of longitudinally extending open channels releasably attachable to the platform. The trajectory selection guide and the multi-lumen device guide are serially interchangeably held by the platform to extend through the port of the platform with a segment thereof residing a distance below the platform.

The system can further include an image processing circuit configured to generate and display a virtual trajectory selection guide member configured as a virtual multi-lumen guide array and aligned with an image of the trajectory guide assembly. The virtual multi-lumen guide array can include a plurality of radially and/or circumferentially spaced apart virtual channels spaced apart about a virtual center channel in a pattern corresponding to positions of the open channels of the multi-lumen device guide. The virtual center channel can be aligned with a center of the open port of the platform.

The system includes at least one drill bit guide that is also releasably and interchangeably extended through the port of the platform and is directly secured to the platform. The at least one drill bit guide includes a rotatable offset guide with a longitudinally extending channel that is offset from an axially extending centerline of the guide and additional guides may include a center guide with a longitudinally extending channel that is centered with an axially extending centerline of the guide; and a rotatable combination guide with a center longitudinally extending channel that is aligned with an axially extending centerline of the guide and a radially offset longitudinally extending channel.

The platform can include directional orientation indicia on an upper surface thereof, wherein the trajectory guide assembly further comprises a pair of arcuate laterally spaced apart arms that hold the platform therebetween and above the base and only two actuators for pitch and roll. The trajectory guide assembly can be devoid of x-y direction actuators.

The virtual multi-lumen guide array and the multi-lumen device guide can have the same number of lumens in a common array configuration.

The trajectory selection guide is a multi-lumen guide array that comprises a plurality of radially and/or circumferentially spaced apart fluid filled lumens spaced apart about a center fluid filled lumen, and wherein the multi-lumen guide array and the multi-lumen device guide have the same number of channels in a common array configuration.

The trajectory selection guide member can be a multi-lumen guide array that has a plurality of spaced apart longitudinally extending fluid filled lumens. The fluid filled channels of the multi-lumen guide array can terminate at a top end under a cap. The multi-lumen device guide can have a top end that is at the same height as the top end of the fluid filled channels.

The plurality of longitudinally extending fluid filled channels can have a common length.

The plurality of fluid filled channels in the multi-lumen guide array and the plurality of open channels in the multi-lumen device guide can be seven

The plurality of longitudinally extending fluid filled channels can have a center channel and adjacently positioned channels residing spaced apart about the center channel.

The multi-lumen device guide array can have orientation indicia corresponding to patient directions of right, left and forward and the platform can have corresponding orientation indicia.

Other embodiments are directed to methods of introducing a device(s) into a subject. The methods include: placing a trajectory frame on a subject, the trajectory frame comprising a base, a yoke attached to the base and a platform attached to the yoke, the platform comprising an open port; inserting a trajectory guide with a single longitudinally extending fluid-filled lumen or a multi-lumen guide array with a plurality of longitudinally extending fluid filled channels through the port and securing the trajectory guide or the guide array directly to the platform; identifying a desired trajectory; removing the trajectory guide or the multi-lumen guide array from the platform; inserting a device guide with multiple open longitudinally extending through channels into the port and securing the device guide to the platform; and introducing at least one device into a channel of the open channels of the device guide and into a body of a subject.

The methods can also include: electronically generating a virtual multi-lumen guide array with a plurality of longitudinally extending parallel virtual channels; electronically aligning the generated virtual multi-lumen guide array with an image of the trajectory frame; and displaying an image with the virtual multi-lumen guide array overlaid on the trajectory frame with the virtual. The virtual multi-lumen guide array can include a plurality of radially and/or circumferentially spaced apart virtual channels spaced apart about a virtual center channel in a pattern corresponding to positions of the open channels of the multi-lumen device guide.

The electronically aligning can be carried out by identifying orientation features of the trajectory guide on the subject in MRI image data and aligning the virtual center channel with a center of the open port of the platform.

According to some embodiments of the present invention, system has a base, a yoke movably mounted to the base and that is rotatable about a roll axis, and a platform movably mounted to the yoke and that is rotatable about a pitch axis. The platform includes a port that can releasably and interchangeably hold a tubular single or multi-lumen fluid filled guide array member and at least one tubular device guide comprising a plurality of longitudinally extending open lumens. The base has a patient access aperture formed therein and is configured to be secured to the body of a patient such that the aperture overlies an opening in the body.

A roll actuator can be operably connected to the yoke and is configured to rotate the yoke about the roll axis. A pitch actuator can be operably connected to the platform and is configured to rotate the platform about the pitch axis.

The base may include a plurality of locations for attachment to a body of a patient via fasteners. In some embodiments, one or more attachment locations may include multiple adjacent apertures configured to receive a fastener therethrough. For embodiments where the frame is configured to be attached to the skull of a patient, the base can be configured to be secured to the skull of a patient such that the patient access aperture overlies a burr hole formed in the patient skull.

According to some embodiments of the present invention, the yoke includes a pair of spaced apart arcuate arms. The platform directly supports the multi-lumen guide array and the multi-lumen device guide and moves along the yoke arcuate arms when rotated about the pitch axis.

The base can include at least one arcuate arm. The yoke engages and moves along the base arcuate arm when rotated about the roll axis.

In some embodiments, the actuators are color-coded such that each different actuator has a respective different color. This allows a user to quickly determine which actuator is the correct one for a particular desired movement of the frame.

The elongated tubular guide extends through the port in the platform and yoke along a Z-direction and includes opposite proximal and distal end portions. The device guide distal end portion is positioned proximate the patient access aperture. The device guide includes a bore therethrough that extends from the proximal end portion to the distal end portion, and the device guide can be configured to removably receive different devices within one or more open bores. The devices may have different sizes and configuration. Exemplary devices include a needle infusion cannula, a tracking device with an array of optical fiducials, a microelectrode drive, a catheter guide, etc.

The at least one tubular device guide can include a multi-lumen device guide with a plurality of parallel longitudinally extending open through-lumens.

In some embodiments of the present invention, the at least one device guide can have a proximal end portion which engages the platform over the port. For example, the device guide proximal end portion may include a detent, or other type of structure (shape and/or component), formed therein, for a quick-release attachment.

The device guide can include a portion having a protrusion configured to engage the detent so as to removably secure the device to the guide via a snap fit. Alternatively, the guide proximal end portion may include a protrusion and the device may include a portion having a detent formed therein that is configured to engage the protrusion so as to removably secure the device to the guide via a snap fit.

The term "quick release," as used herein, means that a technician or other user can quickly (e.g., typically in under about 1 minute or under about 30 seconds) remove a device from the guide with little effort and without requiring tools.

According to some embodiments of the present invention, an interventional method includes affixing a frame with a cooperating single lumen or multi-lumen fluid filled array to the skull of a patient, identifying a desired trajectory, replacing the single lumen or multi-lumen fluid filled array with a device guide.

The method may be carried out in an operating room using a camera based tracking system.

The method may be carried out using images acquired from a CT scanner during the procedure and/or using MRI images.

In some embodiments, such as, for neuro, using both pre-acquired and real time acquired MRI brain images and CT images at one or times during the procedure).

The entire workflow of a patient procedure may be carried out entirely in an MRI suite or in an OR followed by an MRI suite.

It is noted that aspects of the invention described with respect to one embodiment may be incorporated in a different embodiment although not specifically described relative thereto. These and other objects and/or aspects of the present invention are explained in detail below.

### BRIEF DESCRIPTION OF THE DRAWINGS

**FIG. 1A** is a side perspective view of an example stylus of a surgical navigation system that can be used to provide an entry into a patient.
**FIG. 1B** is a schematic side perspective view of the example stylus shown in **FIG. 1A** used to pick an entry point into a skull of a patient.
**FIG. 2A** is a side perspective view of an example centering screw guide that can directly anchor over a selected entry point on a patient, i.e., patient skull, for a twist point entry sequence, according to embodiments of the present invention.
**FIG. 2B** is a top, side perspective view of a screw driver that can be used to secure the centering screw guide shown in **FIG. 2A** to a patient according to embodiments of the present invention.
**FIG. 2C** is a top, side perspective view of the centering screw guide shown in **FIG. 2A** with the screw secured into the skull according to embodiments of the present invention.
**FIG. 2D** is a top perspective view of a centering tool that can cooperate with the centering guide screw to help position a trajectory frame onto the patient according to embodiments of the present invention.
**FIG. 2E** is a top perspective view of the centering tool shown in **FIG. 2D** concentrically positioned about and attached directly onto the centering screw guide according to embodiments of the present invention.
**FIG. 2F** is a top perspective vie of the centering tool that can be directly placed into a divot formed by a burr hole entry (instead of a twist point entry and not requiring a centering guide screw) according to embodiments of the present invention.
**FIG. 2G** is a side perspective view of the centering tool positioned so that a distal end thereof fits directly into the burr hole according to embodiments of the present invention.
**FIG. 3A** is a side perspective view of an example trajectory frame base that can couple to a patient's skull or other target device or anatomy according to embodiments of the present invention.
**FIG. 3B** is a side perspective view of the trajectory frame base aligned over the centering tool shown in **FIGS. 2D** and **2G** according to embodiments of the present invention.
**FIG. 3C** illustrates the trajectory frame base secured to the patient and the centering guide removed according to embodiments of the present invention.
**FIGS. 4A** and **4B** are side perspective views of an example trajectory frame that can be attached to the base shown in **FIG. 3A** according to embodiments of the present invention.
**FIG. 4C** is a partially exploded view of the trajectory frame shown in **FIGS. 4A** and **4B****.**
**FIG. 4D** is a side perspective view of the trajectory frame aligned with the base according to embodiments of the present invention.
**FIG. 4E** is a side perspective assembled view of the trajectory frame and base according to embodiments of the present invention.
**FIG. 5A** a side perspective view of the trajectory frame and base and a navigation stylus adapter that is releasably held by the trajectory frame according to embodiments of the present invention.
**FIG. 5B** is an assembled view of the navigation stylus adapter in the trajectory frame shown in **FIG. 5A****.**
**FIG. 5C** is a top perspective view of a navigation stylus insertable into the adapter shown in **FIG. 5B** according to embodiments of the present invention.
**FIG. 5D** is an assembled view of the components shown in **FIG. 5C****.**
**FIG. 5E** is an enlarged side view of the assembled device shown in **FIG. 5C** illustrating a pitch adjustment actuator for pitch adjustments according to embodiments of the present invention.
**FIG. 5F** is a side perspective view of the assembled device shown in **FIG. 5C** illustrating an example pitch adjusted orientation according to embodiments of the present invention.
**FIG. 5G** is an enlarged side view of the assembled device shown in **FIG. 5C** illustrating a roll adjustment actuator for roll adjustments according to embodiments of the present invention.
**FIG. 5H** is a side perspective view of the assembled device shown in **FIG. 5C** illustrating an example roll-adjusted orientation according to embodiments of the present invention.
**FIG. 6A** is a side perspective view of an example guide array with fluid filled lumens according to embodiments of the present invention.
**FIG. 6B** is a top view of a primary body of the guide array shown in **FIG. 6A****.**
**FIG. 6C** is a side partial exploded view of the guide array shown in **FIG. 6A****.**
**FIG. 6D** is side perspective view of the guide array shown in **FIG. 6A** with internal fluid filled lumen channels shown partially transparent.
**FIG. 7A** is a side perspective view of the guide array aligned with the trajectory frame for assembly thereto according to embodiments of the present invention.
**FIG. 7B** is an enlarged side perspective view of the guide array in the platform of the trajectory guide according to embodiments of the present invention.
**FIG. 7C** is a side perspective assembled view of the components shown in **FIG. 7A****.**
**FIGS. 7D** and **7E** are partial top views of the assembly shown in **FIG. 7C****.**
**FIG. 8** is a side view of a fluid-filled guide array adjacent a multi-lumen guide according to embodiments of the present invention.
**FIGS. 9A-9C** are top, side perspective views of example device guides according to embodiments of the present invention. **Fig. 9B** discloses the device guide of the invention.
**FIG. 10A** is a top, side perspective views of the device guide shown in **FIG.** 9C aligned with the trajectory frame according to embodiments of the present invention.
**FIG. 10B** illustrates the device guide shown in **FIG. 10A** assembled to the trajectory frame.
**FIGS. 10C** and **10D** are top views of the assembly shown in **FIG. 10B** according to embodiments of the present invention.
**FIG. 10E** is a top view of an example user interface providing rotational alignment feedback of a desired orientation of the guide channel to a user according to embodiments of the present invention.
**FIG. 10F** is a partial side perspective view of a drill and drill bit cooperating with the device guide and trajectory frame assembly shown in **FIG. 10B** according to embodiments of the present invention.
**FIG. 10G** is a side perspective view of the drill and drill bit shown in **FIG.** 10F prior to coupling to the device guide according to embodiments of the present invention.
**FIG. 10H** is a top perspective view of the trajectory frame after the device guide shown in **FIG. 10B** is removed with a twist point entry hole made using the drill and drill bit shown in **FIG. 10G****.**
**FIGS. 11A** and **11B** are side perspective views of an example multi-lumen guide with open through channels according to embodiments of the present invention.
**FIG. 11C** is a top view of the device shown in **FIGS. 11A** and **11B** alongside a fluid-filled guide according to embodiments of the present invention.
**FIG. 11D** is a top perspective view of the multi-lumen guide shown in **FIGS. 11A** and **11B** aligned with the trajectory frame according to embodiments of the present invention.
**FIG. 11E** is an assembled view of the components shown in **FIG. 11D****.**
**FIG. 11F** is a side perspective view of an example therapeutic device aligned with the assembled components shown in **FIG. 11F** according to embodiments of the present invention.
**FIG. 11G** illustrates the therapeutic device held by the multi-lumen guide and trajectory frame shown in **FIG. 11F****.**
**FIGS. 12A** and **12B** are partial side perspective assembled views of the assembled components shown in **FIG. 11E** and illustrating a single therapeutic device coupled thereto **(****FIG. 12A****)** and multiple therapeutic devices coupled thereto **(****FIG. 12B****)** according to embodiments of the present invention.
**FIG. 13A** is an enlarged top perspective view of an exemplary grid that can be used to select an entry point according to embodiments of the present invention.
**FIG. 13B** is a top, side perspective view of the grid shown in **FIG. 13A** used with a centering screw guide and screwdriver according to embodiments of the present invention.
**FIG. 13C** illustrates the centering guide coupled to the grid according to embodiments of the present invention.
**FIG. 13D** illustrates a bone screw coupled to the grid according to embodiments of the present invention.
**FIG. 13E** illustrates an enlarged view of the bone screw in position with the grid removed according to embodiments of the present invention.
**FIG. 14A** is an enlarged partial section view of an example targeting cannula according to embodiments of the present invention.
**FIG. 14B** **is** a side perspective view of the targeting cannula shown in **FIG. 14A** aligned with the trajectory frame shown in **FIG. 4E** according to embodiments of the present invention.
**FIGS. 14C** and **14D** are side perspective assembled views of the components shown in **FIG. 14B****.**
**FIGS. 14E** and **14F** are side views illustrating example pitch adjustments using the targeting cannula and trajectory frame shown in **FIG. 14B****.**
**FIGS. 14G** and **14H** are side views illustrating example roll adjustments using the targeting cannula and trajectory frame shown in **FIG. 14B****.**
**FIG. 15A** is a schematic illustration of a surgical navigation system that uses a virtual guide array according to embodiments of the present invention.
**FIG. 15B** is a top view of a trajectory frame with a multi-lumen guide according to embodiments of the present invention.
**FIG. 16** is a flow chart of exemplary actions that can be used for a medical procedure according to embodiments of the present invention.
**FIG. 17** is a block diagram of a data processing system according to embodiments of the present invention.

### DETAILED DESCRIPTION

The present invention now is described more fully hereinafter with reference to the accompanying drawings, in which some embodiments of the invention are shown. This invention may, however, be embodied in many different forms and should not be construed as limited to the embodiments set forth herein; rather, these embodiments are provided so that this disclosure will be thorough and complete, and will fully convey the scope of the invention as defined by the claims to those skilled in the art.

Like numbers refer to like elements throughout. In the figures, the thickness of certain lines, layers, components, elements or features may be exaggerated for clarity. The terms "Fig." and "FIG." may be used interchangeably with the word "Figure" as abbreviations thereof in the specification and drawings.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising", when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on", "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is inverted, elements described as "under" or "beneath" other elements or features would then be oriented "over" the other elements or features. Thus, the exemplary term "under" can encompass both an orientation of "over" and "under". The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

The term "about", as used herein with respect to a value or number, means that the value or number can vary by +/- twenty percent (20%).

The term "MRI visible" means that a device is visible, directly or indirectly, in an MRI image. The visibility may be indicated by the increased SNR of the MRI signal proximate to the device (the device can act as an MRI receive antenna to collect signal from local tissue) and/or that the device actually generates MRI signal itself, such as via suitable hydro-based coatings and/or fluid (typically aqueous solutions) filled channels or lumens.

The term "MRI compatible" means that a device is safe for use in an MRI environment and/or can operate as intended in an MRI environment without generating MR signal artifacts, and, as such, if residing within the high-field strength region of the magnetic field, is typically made of a non-ferromagnetic MRI compatible material(s) suitable to reside and/or operate in a high magnetic field environment.

The term "high-magnetic field" refers to field strengths above about 0.5T (Tesla), typically above 1.0T, and more typically between about 1.5T and 10T.

The term "targeting cannula" refers to an elongate device, typically having a substantially tubular body that can be oriented to provide positional data relevant to a target treatment site and/or define a desired access path orientation or trajectory. At least portions of a targeting cannula contemplated by embodiments of the invention can be configured to be visible in an MRI image, thereby allowing a clinician to visualize the location and orientation of the targeting cannula in vivo relative to fiducial and/or internal tissue landscape features.

The term "cannula" refers to an elongate device that can be associated with a trajectory frame that attaches to a patient, but does not necessarily enter the body of a patient.

The term "imaging coils" refers to a device that is configured to operate as an MRI receive antenna. The term "coil" with respect to imaging coils is not limited to a coil shape but is used generically to refer to MRI antenna configurations, loopless, looped, etc., as are known to those of skill in the art. The term "fluid-filled" means that the component includes an amount of the fluid but does not require that the fluid totally, or even substantially, fill the component or a space associated with the component. The fluid may be an aqueous solution, MR contrast agent, CT contrast material or any material that generates a signal in the imaging modality used.

The term "two degrees of freedom" means that a trajectory frame described herein allows for at least translational (swivel or tilt) and rotational movement over a fixed site, which may be referred to as a Remote Center of Motion (RCM).

The terms "ACPC coordinate space" or "AC-PC orientation" refers to a right-handed coordinate system defined by anterior and posterior commissures (AC, PC) and Mid-Sagittal plane points, with positive directions corresponding to a patient's anatomical Right, Anterior and Head directions with origin at the mid-commissure point.

Embodiments of the present invention can be configured to guide and/or place diagnostic or interventional devices and/or therapies to any desired internal region of the body or object using MRI and/or in an MRI scanner or MRI interventional suite or using other image guided systems not requiring an MRI system or suite.

The object can be any object, and may be particularly suitable for animal and/or human subjects. Some embodiments can be sized and configured to place implantable DBS leads for brain stimulation, typically deep brain stimulation. Some embodiments can be configured to deliver tools or therapies that stimulate a desired region of the sympathetic nerve chain. Other uses inside or outside the brain include stem cell placement, gene therapy or drug delivery for treating physiological conditions. Some embodiments can be used to treat tumors. Some embodiments can be used for RF ablation, laser ablation, cryogenic ablation, etc.

In some embodiments, the trajectory frame and/or interventional tools can be configured to facilitate high resolution imaging via integral intrabody imaging coils (receive antennas), high intensity focused ultrasound (HIFU), and/or the interventional tools can be configured to stimulate local tissue, which can facilitate confirmation of proper location by generating a physiologic feedback (observed physical reaction or via fMRI).

Some embodiments can be used to deliver bions, stem cells or other target cells to site-specific regions in the body, such as neurological target sites and the like. In some embodiments, the systems deliver stem cells and/or other cardio-rebuilding cells or products into cardiac tissue, such as a heart wall via a minimally invasive image guided procedure, while the heart is beating (i.e., not requiring a non-beating heart with the patient on a heart-lung machine). Examples of known stimulation treatments and/or target body regions are described in U.S. Patent Nos. 6,708,064; 6,438,423; 6,356,786; 6,526,318; 6,405,079; 6,167,311; 6539,263; 6,609,030 and 6,050,992.

Generally stated, some embodiments of the invention are directed to interventional procedures and provide interventional tools and/or therapies that may be used to locally place interventional tools or therapies in vivo to site-specific regions using an image guided system. The interventional tools can be used to define a trajectory or access path to an in vivo treatment site. Some embodiments of the invention provide interventional tools that can provide positional data regarding location and orientation of a tool in 3-D space with a visual confirmation on an image. Embodiments of the invention may provide an integrated system or trajectory frames and components that can be used with one or more of commercially available conventional image guided systems that may allow physicians to place interventional devices/leads and/or therapies accurately.

Some embodiments configure devices so that they are compatible with several imaging modalities and/or image-guided systems.

For MRI uses, the systems may allow for shorter duration procedures over conventional systems (typically under six hours for DBS implantation procedures, such as between about 1-5 hours).

In some embodiments, a pre-operative image such as an MRI image can be used to visualize (and/or locate) a therapeutic region of interest inside the brain or other body locations. During surgery, the MRI or other pre-operative image can be used to visualize (and/or locate) an interventional tool or tools that will be used to deliver therapy and/or to place a chronically implanted device that will deliver therapy.

Embodiments of the invention provide devices and an operational sequence of a procedure that can be initiated in a first operating room then completed in a second operating room such as an MRI suite according to some embodiments of the present invention.

The same trajectory frame 100 can serially releasably hold a trajectory guide member that can have at least one elongate, longitudinally extending, fluid filled lumen, i.e., a single fluid filled lumen or may be configured as a multi-lumen fluid filled guide array, and interchangeable elongate device guides which can have one or multiple through/open lumens as will be discussed below. In some embodiments, an entire surgical procedure can be carried out in the Operating Room (OR) not requiring the use of an MRI suite using some of the devices shown.

In some embodiments, the three-dimensional data produced by a CT-guided and/or MRI-guided interventional system regarding the location of the therapeutic region of interest and the location of the interventional tool can allow the system and/or physician can make positional adjustments to the interventional tool so as to align the trajectory of the interventional tool with the region of interest, so that when inserted into the body, the interventional tool will intersect with the therapeutic region of interest.

In some embodiments, a camera based tracking system can be used.

The systems can have a hardware component(s) and a software component(s). In some embodiments, the hardware component includes a camera and workstation that can be used for many applications such as cranial, spine, orthopedic, ENT. There can be different software packages or modules for each system and/or for each application.

When the imaging system and/or the camera based image guided system confirms alignment is proper, the interventional tool aligned with the therapeutic region of interest, an interventional probe can be advanced, such as through an open lumen inside of the interventional tool, so that the interventional probe follows the trajectory of the interventional tool and proceeds to the therapeutic region of interest. It should be noted that the interventional tool and the interventional probe may be part of the same component or structure. A sheath may optionally form the interventional tool or be used with an interventional probe or tool.

In particular embodiments, using MRI in combination with local or internal imaging coils and/or MRI contrast material that may be contained at least partially in and/or on the interventional probe or sheath, the location of the interventional probe within the therapeutic region of interest can be visualized on a display or image and allow the physician to either confirm that the probe is properly placed for delivery of the therapy (and/or placement of the implantable device that will deliver the therapy) or determine that the probe is in the incorrect or a non-optimal location. Assuming that the interventional probe is in the proper desired location, the therapy can be delivered and/or the interventional probe can be removed and replaced with a permanently implanted therapeutic device at the same location.

Although described and illustrated herein with respect to the brain and the insertion of deep brain stimulation leads, it is understood that embodiments of the present invention may be utilized at other portions of the body and for various other types of procedures.

The image-guided system can be used for MRI and/or non-MRI image guided systems.

The trajectory frame and some or all of its cooperating components may be configured to be compatible for use in MRI and CT and/or camera based image guided systems." To be clear, the term "image guided system" is used generally to refer to surgical navigation systems that include displays with patient images (which may be acquired before a surgery and/or at defined points during a surgery to confirm location) but does not require a continuous series of images from an imaging modality, such as a CT or MRI scanner, during the surgery.

In some embodiments, the system can include or work with a trajectory guide software module that can be an off-the-shelf module provided with conventional image guided systems that does not require any (or insignificant) modification. Examples of known commercial systems with trajectory guide software modules for camera based image guided systems that can be used with configurations of the trajectory frames and cooperating components include, for example systems from Brainlab, Inc., Stryker Medical and Medtronic Inc.

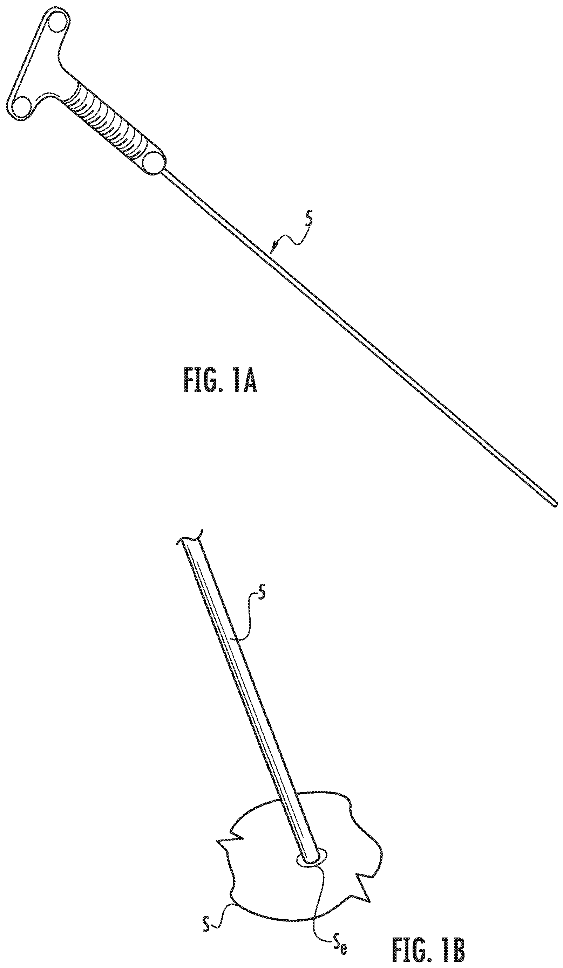

Referring to **FIGS. 1A** and **1B****,** a navigation stylus **5** can be used to find a pre-planned trajectory and intrabody entry point, such as an entry point **Se** into a skull S of a patient. The stylus **5** is shown by way of example only and can have other shapes and configurations. The stylus **5** can be part of an OR navigation system used outside an MRI suite. There are two primary (or at least preferred) options for a surgeon to use to create an entry point **Se** into a brain through a skull. Option 1 is to use a twist point drill to create a small access hole typically in a range of about 2 mm to about 6.0 mm, such as about 3.4 mm, about 4.5 mm and about 6.0 mm. Option 2 is to create a larger burr hole such as a burr hole in a range of bout 10-mm to about 15 mm, such as about 14 mm.

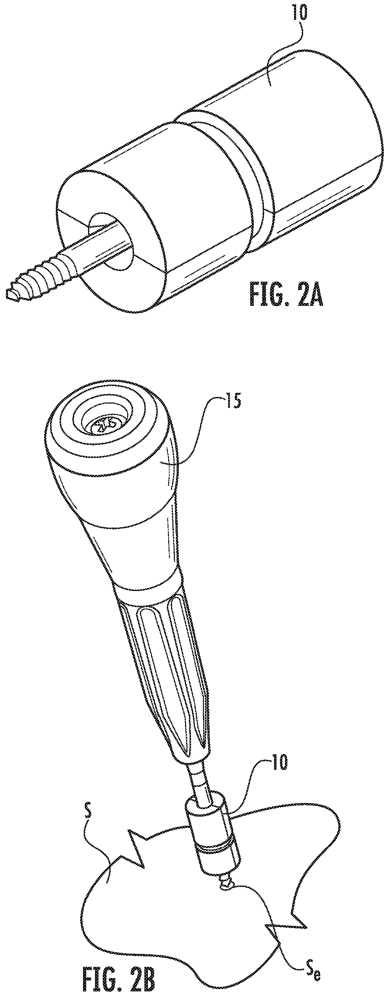

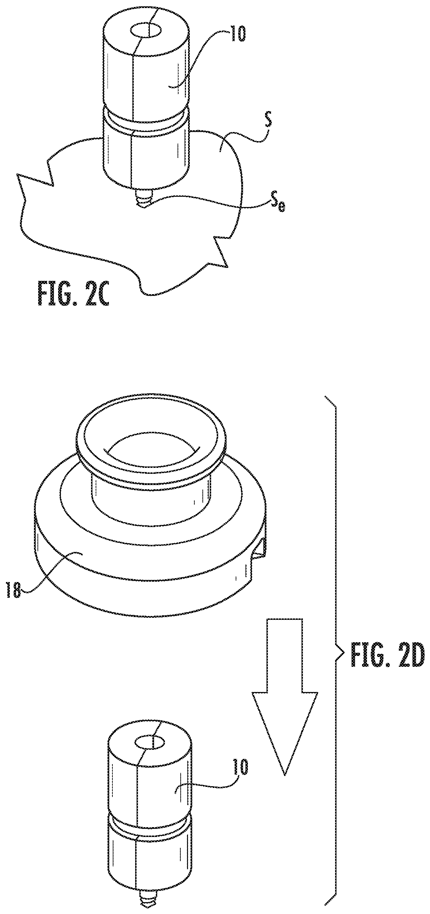

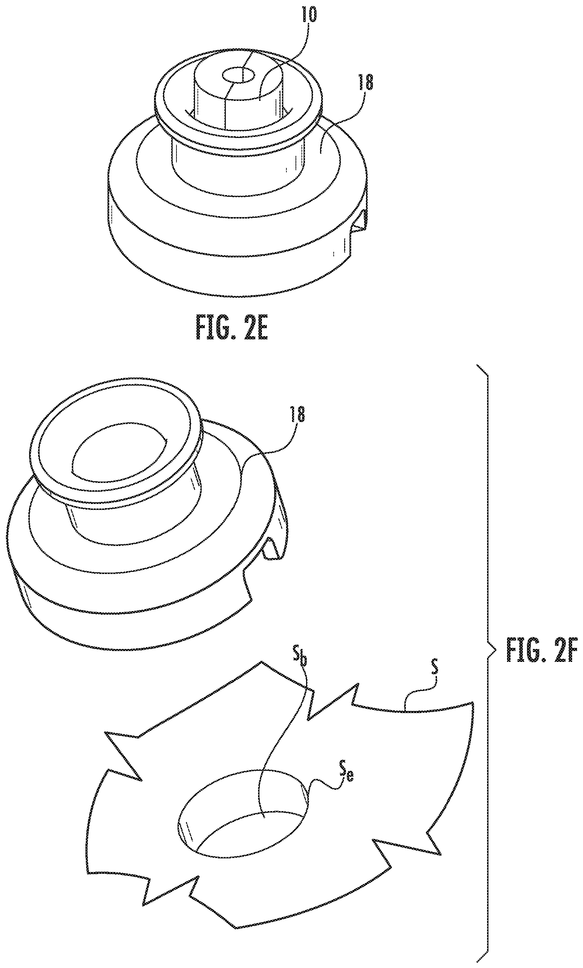

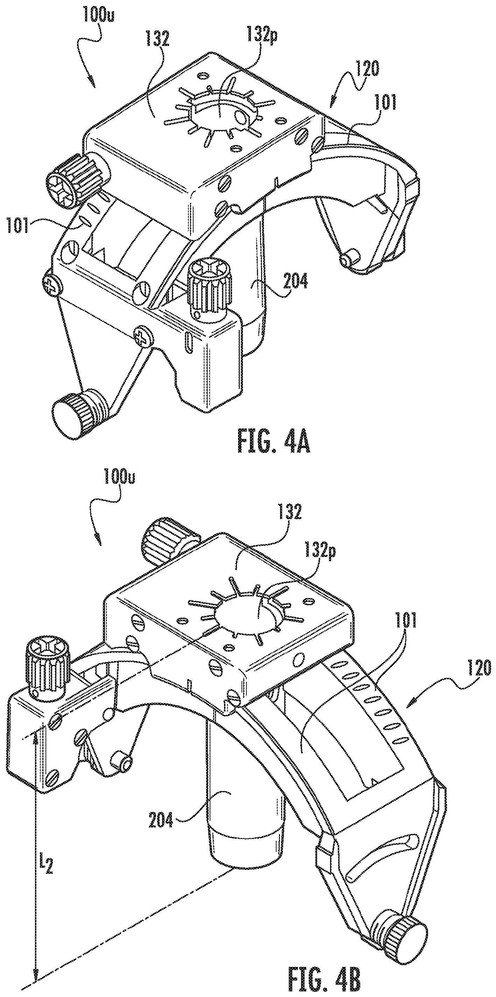

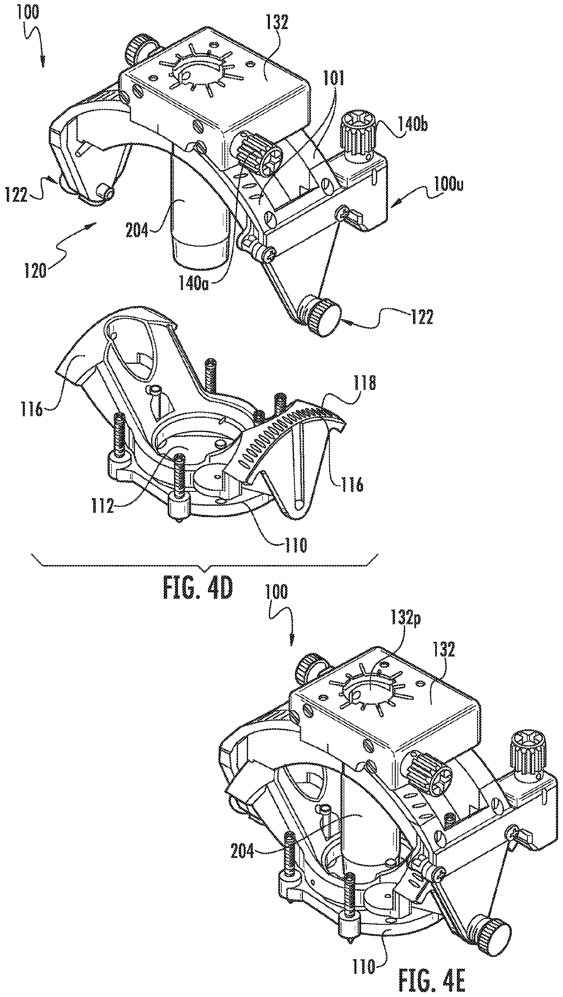

**FIGS. 2A-2E** illustrate the use of a centering screw guide **10** that can be directly attached to a patient at the selected entry point **Se** via a screw driver **15** for Option 1. **FIG. 2D** illustrates a centering tool **18** can be posited onto the centering screw guide 10. The centering tool **18** can enter a base **110** that can support a trajectory frame **100 (****FIG. 4A****).** The term "trajectory frame" is used interchangeably with "trajectory guide assembly." The centering tool **18** can fit concentrically onto and over the centering screw guide **10** as shown in **FIG. 2E****.**

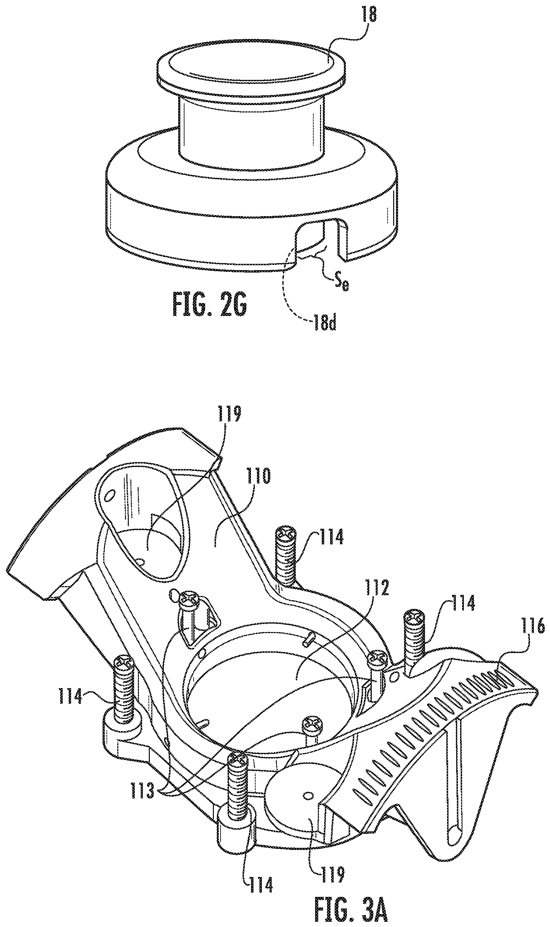

**FIGS. 2F** and **2G** illustrate that the centering tool **18** can fit directly into a burr hole **Sb** formed by Option 2. The burr hole **Sb** can have a diameter in a range of about 10 mm - 15 mm, such as about 14 mm, and can be formed through the skull based on a smaller divot made by the navigation stylus **5 (****FIG. 1A****).** A distal end **18d** of the centering tool **18** can fit directly into the burr hole **Sb.**

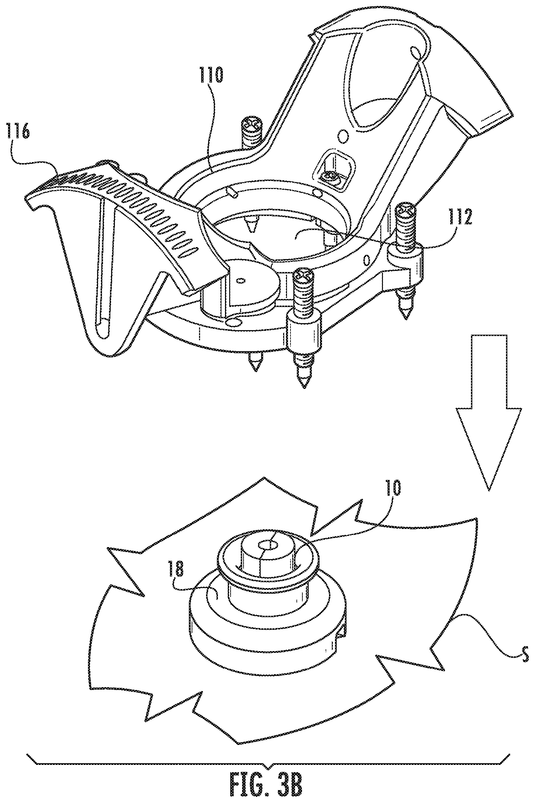

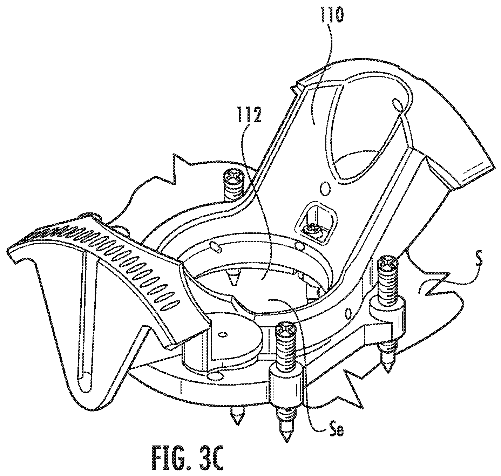

**FIG. 3A** illustrates an example trajectory frame base **110** that can be used to anchor to the patient's skull. The base **110** can be a scalp mount base with a plurality of bone screws **113** and a plurality of stand-off pins **114.** This configuration uses a minimal incision over the entry point Se to create the access. Other trajectory frame bases can be used including, for example, a skull mount base which uses a larger incision and can mount directly to the skull but requires the scalp to be retracted for the direct skull attachment (not shown). **FIG. 3B** illustrates the base aligned with the centering tool **18** that is attached to the centering screw guide **10.** A distal end of the base **110** can have a port **112** that fits concentrically about the centering tool **18.** As shown in **FIG. 3C****,** once the base **110** is secured to the patient, the centering tool **18** can be removed (as well as the centering screw guide 10, if used).

Referring to **FIGS. 4A-4E****, 7A-12B,** a trajectory frame **100** is shown. The upper portion **100u** of the trajectory frame **100** can be attached to the base **110** after the base **110** is secured to the patient. The base **110** can be affixed to the trajectory frame **100** via fixation screws **122 (****FIG. 4D****).**

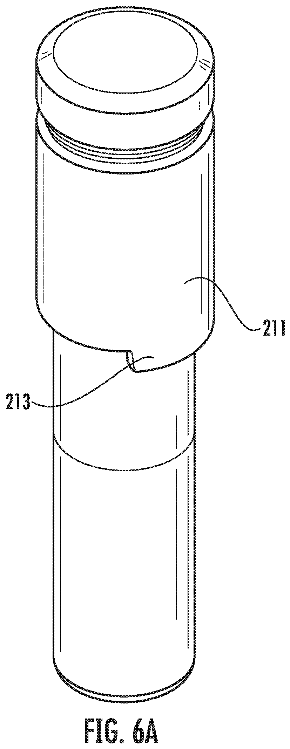

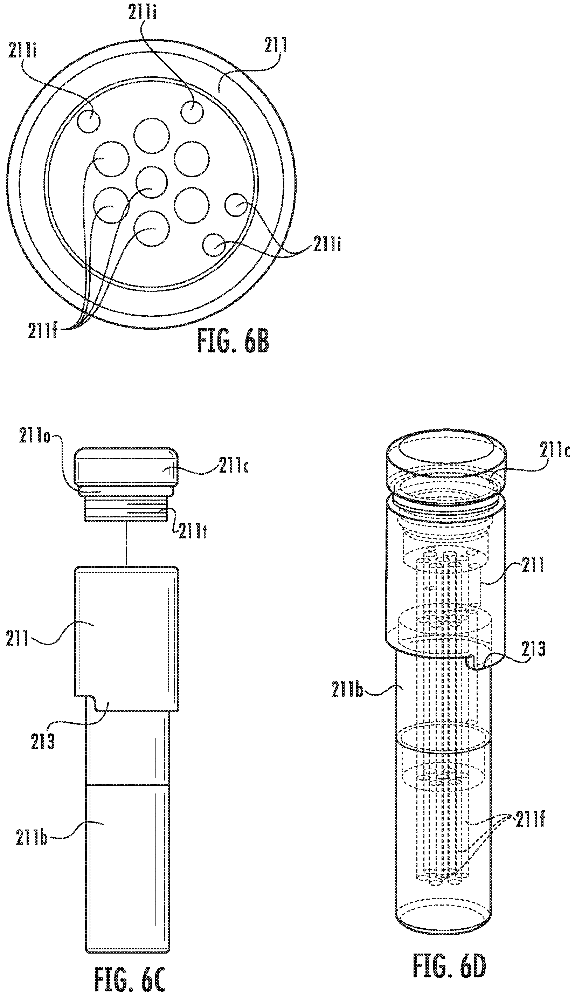

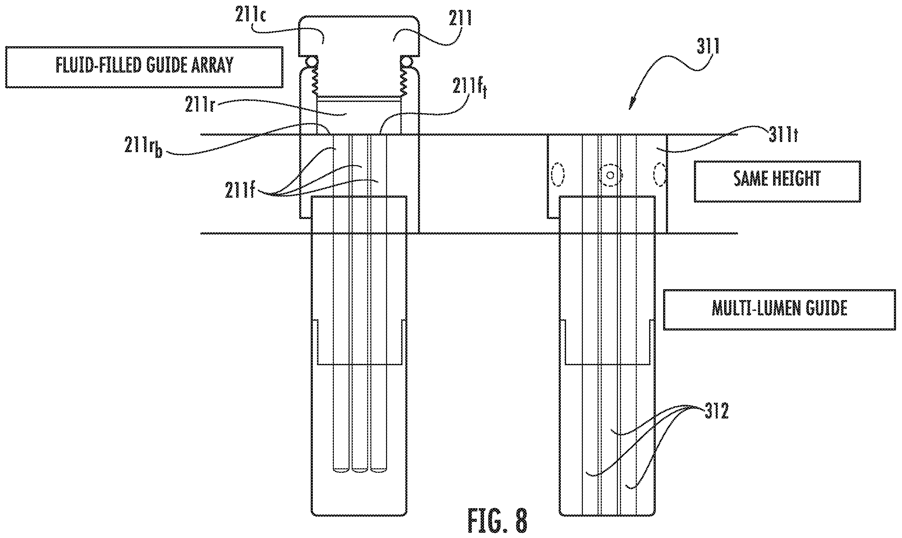

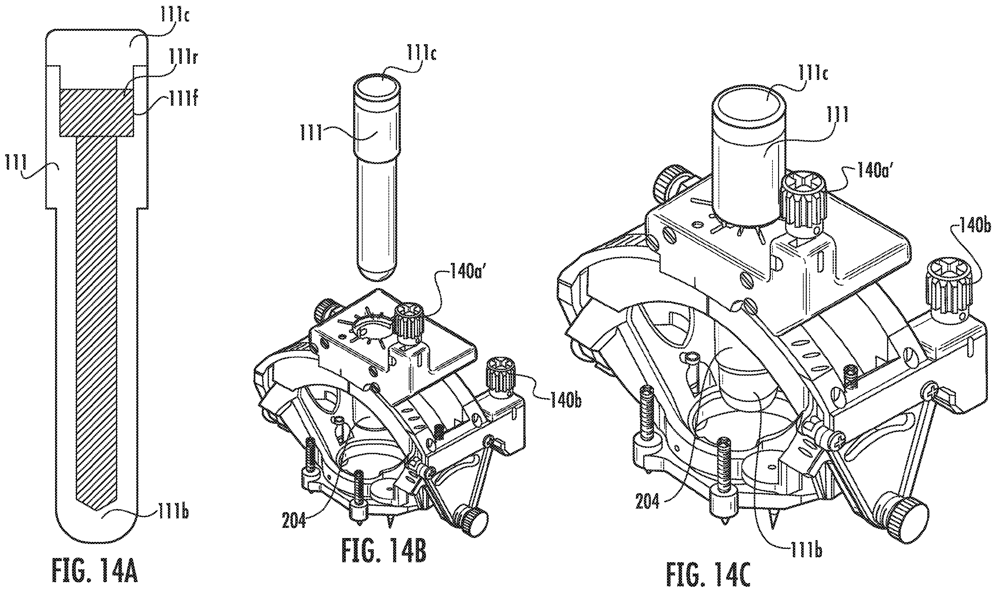

Generally stated, the trajectory frame **100** may be configured to releasably and interchangeably (serially) hold different devices such as, for example, a fluid-filled single lumen guide **111 (****FIGS. 14A-14C****)** which may also be referred to as a "targeting cannula" and/or a multi-lumen guide array **211 (FIGS. 6A-6E)** and at least one device guide **311 (****FIG. 8****).** The guides **111** and **211** can also be referred to as a trajectory selection guide member.

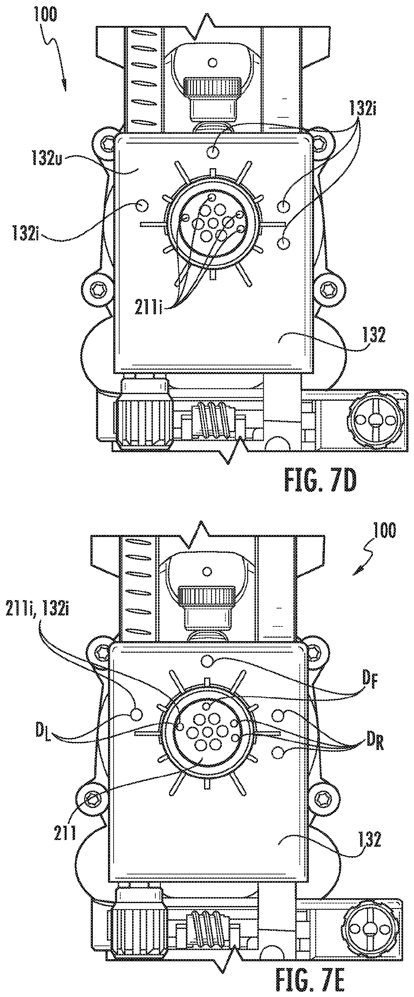

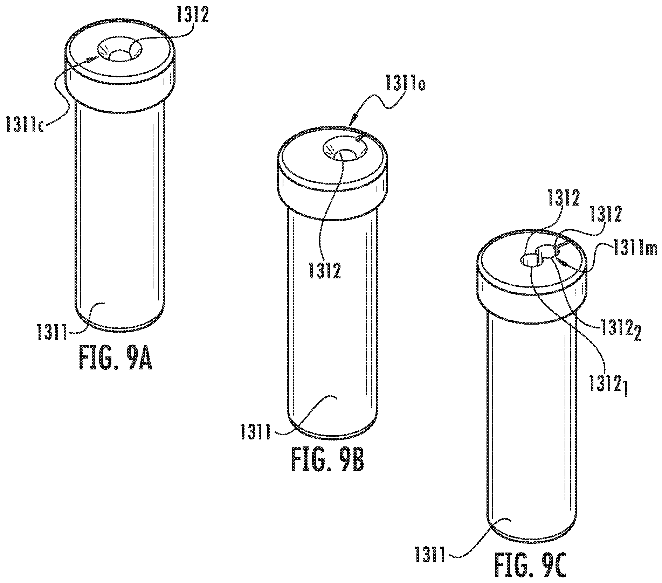

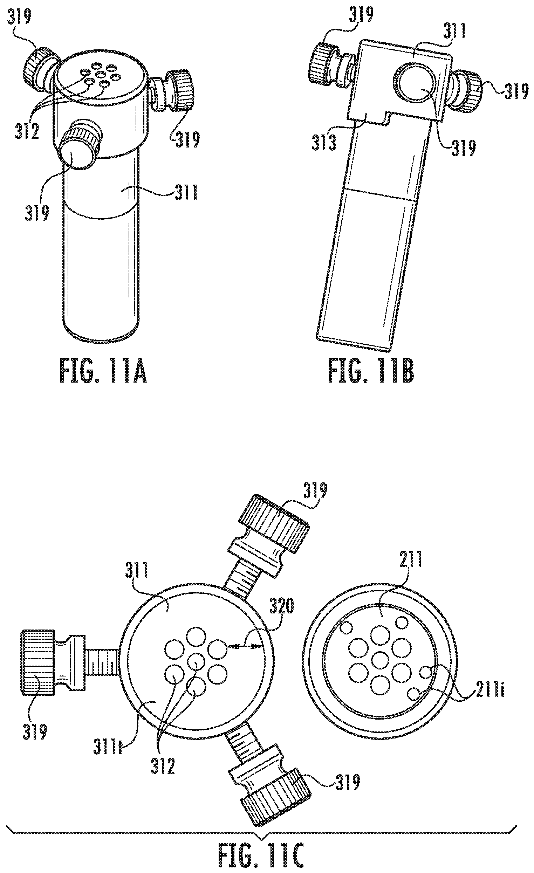

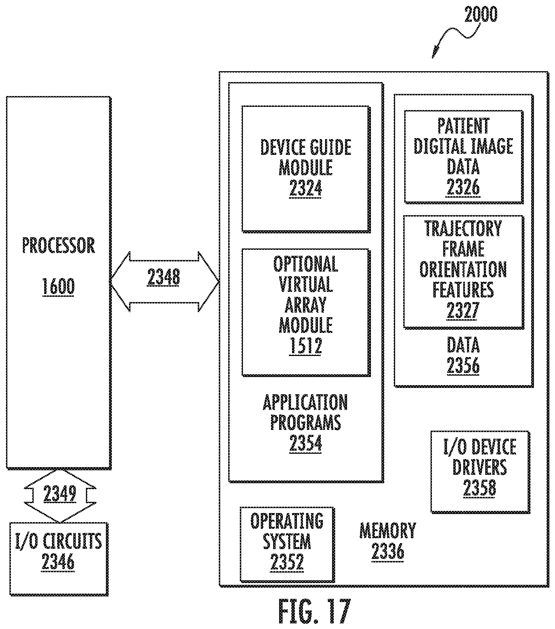

Referring to **FIGS. 4A-4E****,** the trajectory frame **100** can include a tubular member **204,** such as a tower or column, that is held by the platform and that extends a distance below the platform **132.** The platform **132** can be planar and have an open port **132p** that removably and interchangeably (serially) receives one or more of the single fluid-filled lumen guide **111 (FIGS. 17A-17C),** the fluid-filled guide array **211** and one or more different device guides **311 (****FIGS. 11A-12B****)** and optionally one or more drill guides **1311 (****FIG. 9****)** that have open lumens. The planar platform **132** can be rectangular and held by arcuate arms **101** of the trajectory frame **100.** The planar platform **132** can have orientation indicia **132i (****FIG. 7E****)** on a top surface thereof. In some embodiments, the device guide **311** can have the same number and configuration of lumens as the fluid-filled guide array **211 (****FIGS. 8****,** **11C).**

The tubular member **204** can define a Z-direction along its longitudinal axis relative to the X-Y plane of the platform **132** (which does not include an X-Y table).

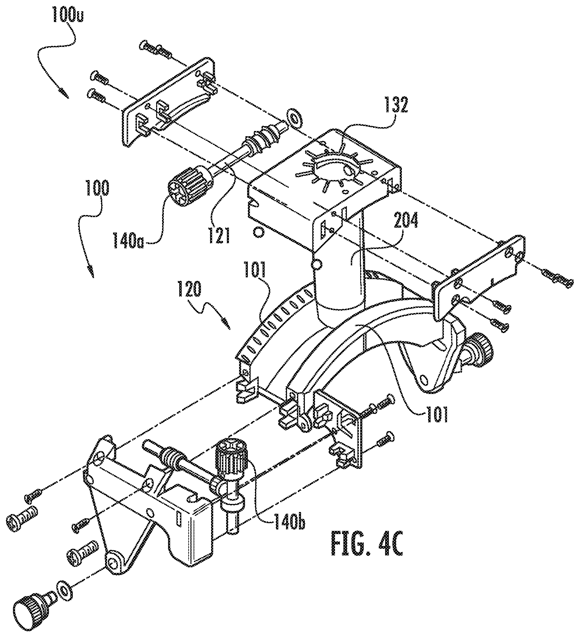

Referring to **FIGS. 4C** and **4D****,** the yoke **120** is movably mounted to the base **110** and is rotatable about a roll axis. A roll actuator **140b** is operably connected to the yoke **120** and is configured to rotate the yoke **120** about the roll axis. In some embodiments, the yoke **120** has a range of motion about the roll axis of about seventy degrees (70°). However, other ranges, greater and lesser than 70°, are possible, e.g., any suitable angle typically between about 10°- 90°, 30°- 90°, etc. The illustrated platform **132** is movably mounted to the yoke **120** and is rotatable about a pitch axis. A pitch actuator **140a** is operably connected to the platform **132** and is configured to rotate the platform **130** about the pitch axis. In some embodiments, the platform **132** has a range of motion about the pitch axis of about seventy degrees (70°). However, other ranges, greater and lesser than 70°, are possible, e.g., any suitable angle typically between about 10°- 90°, 30°- 90°, etc.

The base **110** also includes a pair of spaced apart arcuate arms **116,** as illustrated in **FIG. 4D****.** The yoke **120** engages and moves along the base arcuate arms **116** when rotated about the roll axis. In the illustrated embodiment, one of the base arcuate arms **116** includes a thread pattern **118** formed in (e.g., embossed within, machined within, etc.) a surface **116a** thereof. However, in other embodiments, both arms **116** may include respective thread patterns.

One or both actuators **140a, 140b** can include a rotatable worm gear (i.e., worm **121,** **FIG. 4C****)** with teeth that are configured to engage a thread pattern. As the worm gear is rotated, the teeth travel along the thread pattern in the arcuate arm surface.

Referring to **FIG. 4D****,** for example, the trajectory frame **100** includes a base **110,** a yoke **120** with the arcuate arms **101,** the platform **130,** and only two actuators **140a-140b,** which are pitch and roll actuators. No x-y actuators are provided in this embodiment. The base **110** has a patient access aperture **112** formed therein, as illustrated. The base **110** is configured to be secured (directly or indirectly) to the skull of a patient such that the patient access aperture **112** overlies a burr hole in the patient skull. The patient access aperture **112** can be centered over the burr hole via the removable centering device **18** as discussed above **(****FIG. 2D****).**

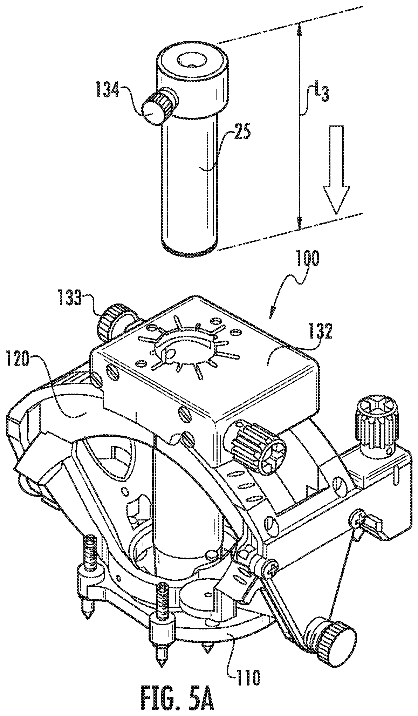

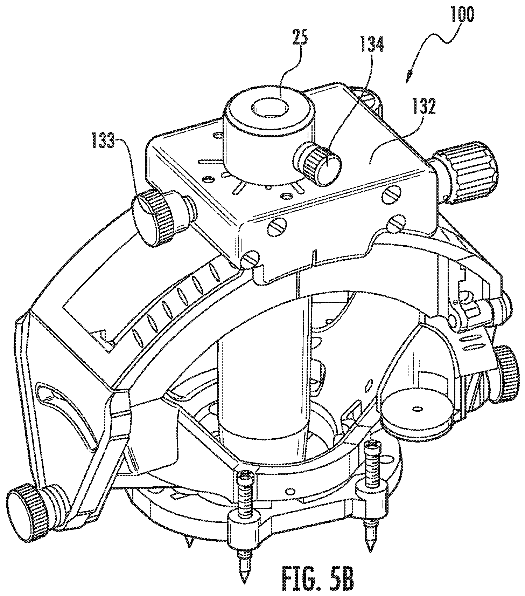

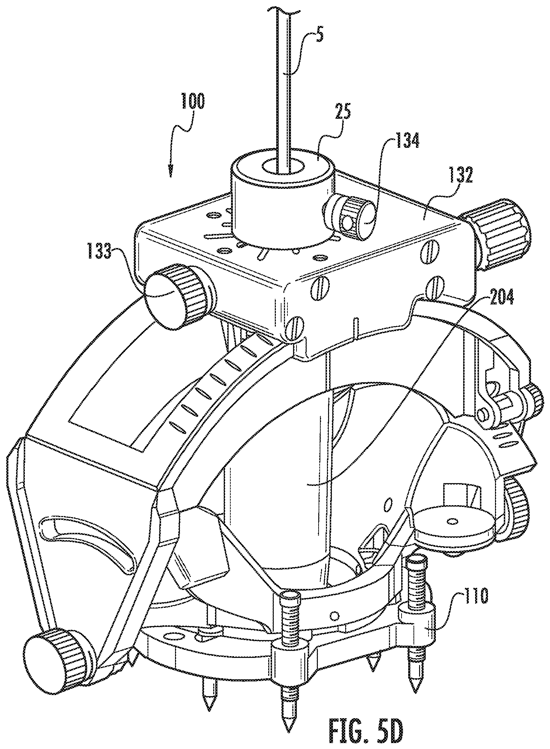

Referring to **FIGS. 5A-5H****,** the trajectory frame **100** can releasably hold a navigation stylus adapter **25.** A fixation screw **133** in the platform **132** can tighten against the adapter **25.** The navigation stylus adapter **25** can secure the navigation stylus **5** such that it is concentrically aligned with the port **132p** and used to make trajectory adjustments. The stylus **5** can be inserted into the stylus adapter **25** until it bottoms out inside the adapter **25** (i.e., it does not extend outside the bottom end of the adapter **25).** A fixation screw **134** on an upper end portion of the adapter **5** can then be tightened against the stylus **5.**

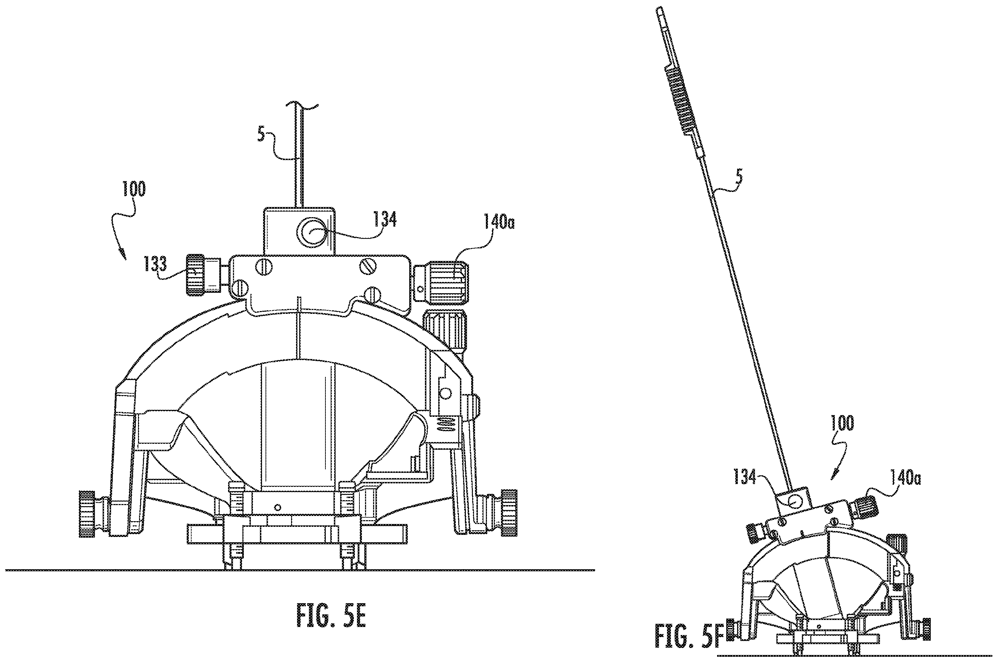

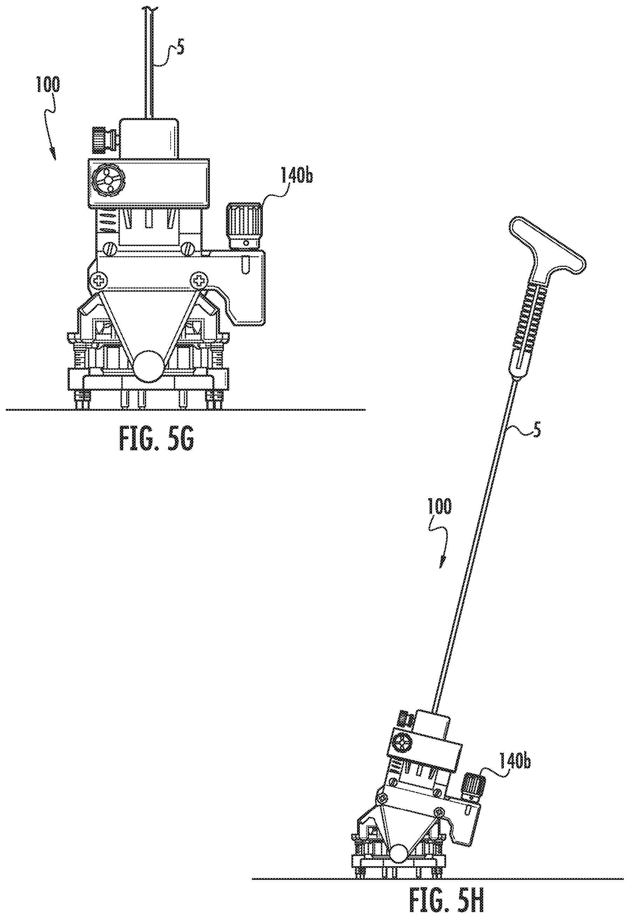

**FIGS. 5E** and **5F** illustrate pitch adjustments (which can be clockwise or counterclockwise) via pitch actuator **140a** and **FIGS. 5G** and **5H** illustrate roll adjustments via roll actuator **140b** (which can be clockwise or counterclockwise).

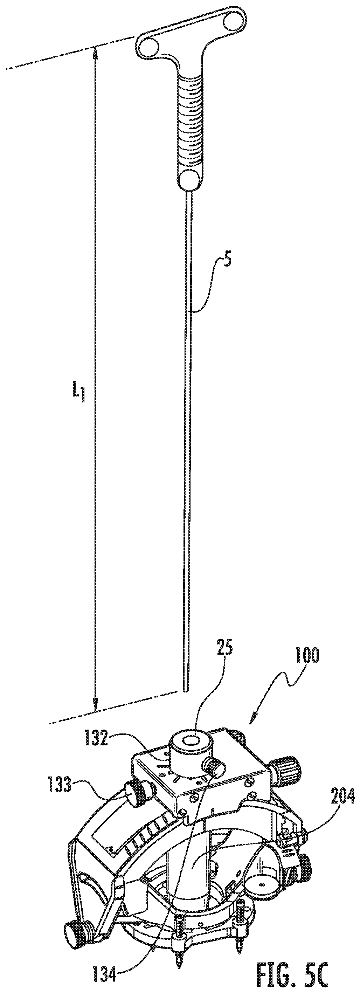

Referring to **FIGS. 4B****,** **5A** and **5C****,** the stylus **5** can have a length **L₁ (****FIG. 5C****)** that is much greater than the length **L₂** of the tubular (support) member **204** and the stylus adapter **25.** The tubular member **204** can have a length **L₂ (****FIG. 4B****)** that is greater than the length **L₃ (****FIG. 5A****)** of the stylus adapter **25. L₁** can be 3X-20X greater than **L₂,** in some embodiments, more typically 4X-8X greater.

Once the trajectory alignment is complete (the trajectory defined by the trajectory guide frame **100** and stylus **5** are approved by a surgeon), the patient can be moved from the OR to a surgical room which may be an MRI suite for further steps in a procedure/further treatment.

Referring to **FIGS. 6A-6E,** after a trajectory is selected/set, a CT and/or MRI visible fluid filled guide **211** can be secured to the trajectory frame **100** (after stylus adapter **25,** where used, is removed) and be used to pick a path to the target. In some embodiments, the fluid-filled guide **211** can provide a plurality of selectable paths, each path associated with a straight linear and (MR visible) fluid filled lumen **211f** defined by the guide array **211** to give a surgeon multiple options for selecting a safe path to the desired target. In other embodiments, the fluid filled guide can provide a single fluid filled lumen. The multiple paths can allow a surgeon to select a path from one of the plurality of paths associated with the lumens **211f to** counter-act any mounting errors. The fluid-filled guide **211** can have a cap **211c** threadably or otherwise sealably attached to the primary body **211b** of the guide array. The cap **211c** can include an O-ring **211o** to prevent or inhibit leakage of fluid from the lumens **211f,** once filled with an MRI and/or CT visible fluid.

As shown in **FIGS. 6D** and **6E,** there can be a plurality of closely spaced apart fluid filled lumens **211f,** typically in a range of 4-10, shown as 7. Where multiple fluid filled lumens are provided, the guide **211** can be referred to as a "guide array." The guide **211** can include channels as directional indicators **211i,** shown as patient left **D_{L}** (single), forward **D_{F}** (single), patient right **D_{R}** (dual). The directional indicator channels **211i** can have a more shallow depth, shorter length and/or different (i.e., smaller or larger) cross-sectional size than the one or more fluid filled lumens **211f.**

As discussed above, the fluid filled guide **211** can have orientation indicia **211i** as shown in **FIGS. 7D** and **7E****,** shown with four spaced apart indicia with two orientation channels **211i** being closer than the other two. The platform **132** can have visual orientation indicia **132i** that corresponds to that of the guide **211i,** shown as **D_{L}, DF** and **D_{R}.** The indicia **211i, 132i** can include two adjacent indicia for a patient right directional indicator, one for a patient left directional indicator and one for a forward directional indicator, for example. The orientation indicia **132i** on the platform **132** can be painted, coated or otherwise provided with color-coded markings on an upper surface **132u** of the platform **132** that can help a user to align the guide **211** and/or identify a channel and/or path selection.

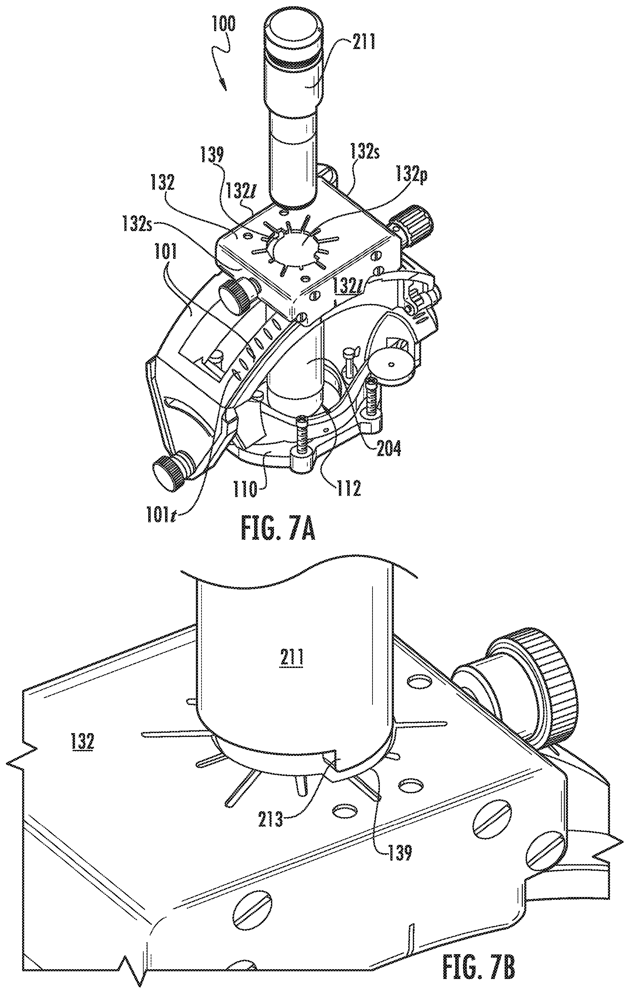

Referring to **FIG. 7A****,** for example, the open port **132p** can optionally be off center over the arcuate arms **101** to reside closer to one short side **132s** of the platform **132** and can be centered side to side with respect to the long sides **1321** of the platform.

Referring to **FIG. 7A****,** the platform **132** engages and moves along the yoke arcuate arms **101** when rotated about the pitch axis. In the illustrated embodiment, one of the yoke arcuate arms **101** includes a thread pattern **101t** formed in (e.g., embossed within, machined within, etc.). However, in other embodiments, both arms **101** may include respective thread patterns.

**FIGS. 7A-7E** show the guide **211** attached to the trajectory frame/guide **100.** The guide array **211** can be locked and secured to the trajectory frame via the fixation screw **133** of the platform **132** that was discussed above as used to secure the stylus adapter **25.** As shown in **FIGS. 7A** and **7B****,** the guide **211** can have an external alignment feature **213** that engages a mating alignment feature **139** in the platform **132** of the trajectory frame **100** to facilitate correct orientation upon assembly. As shown, the alignment feature **213** is a projecting ledge while the mating feature is an enlarged perimeter segment of the port **132.** However, other affirmative alignment configurations may be used.

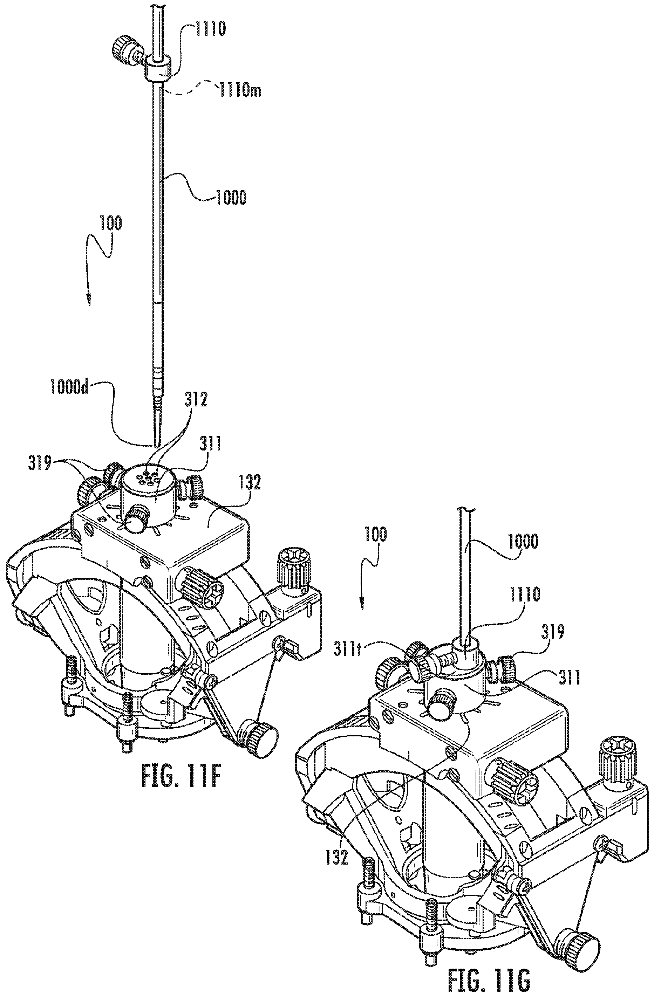

Referring to **FIG. 8****,** the device guide **311** can have lumens **312** that are open channels and a height H that is less than that of the fluid filled guide **211.** The fluid filled lumens **211f** can have a top **211ft** that is under a cap **211c** (which can also be referred to as a bottom of a reservoir under the cap **211c)** and can be at the same height dimension H as the top **311t** of the multi-lumen device guide **311.** The fluid filled guide **211** can be used to identify and/or select a trajectory to the intrabody target. This same trajectory can be used for introducing a medical device **(1000,** **FIG. 11F****)** using the device guide **311,** which replaces the multi-lumen guide array **211** held by the trajectory frame **100.** A line can be electronically drawn from the intrabody target up a desired trajectory along one of the fluid filled lumens **211f to** the top of the fluid filled channel **211ft** of the fluid-filled guide array **211.** The distance between the intrabody target and a bottom of the reservoir/top of the fluid filled channel **211fₜ** can be a device insertion depth of a medical device **1000 (****FIG. 11F****).**

Once the fluid-filled guide **211** is in position in the trajectory frame **100,** a clinician can perform an MRI scan that encompasses an image volume of the trajectory frame **100** and a desired intrabody target. The fluid filled guide channel(s)/lumen(s) **211f will** be bright lines in an MRI image. A surgeon can select a fluid filled lumen **211f** that most closely aligns or matches the desired insertion path. The clinician (i.e., surgeon) can electronically cause the surgical system to programmatically calculate and/or measure a device insertion depth using measurement software. That is, a line can be drawn from the target up the desired trajectory along a selected fluid filled lumen(s) **211f,** to the bottom of the reservoir **211r_{b}** and/or top of the fluid filled lumen **211ft.** The distance between the target and the bottom of the reservoir **211rb/**top of the fluid filled lumen **211fₜ.** can be used to calculate the device insertion depth.

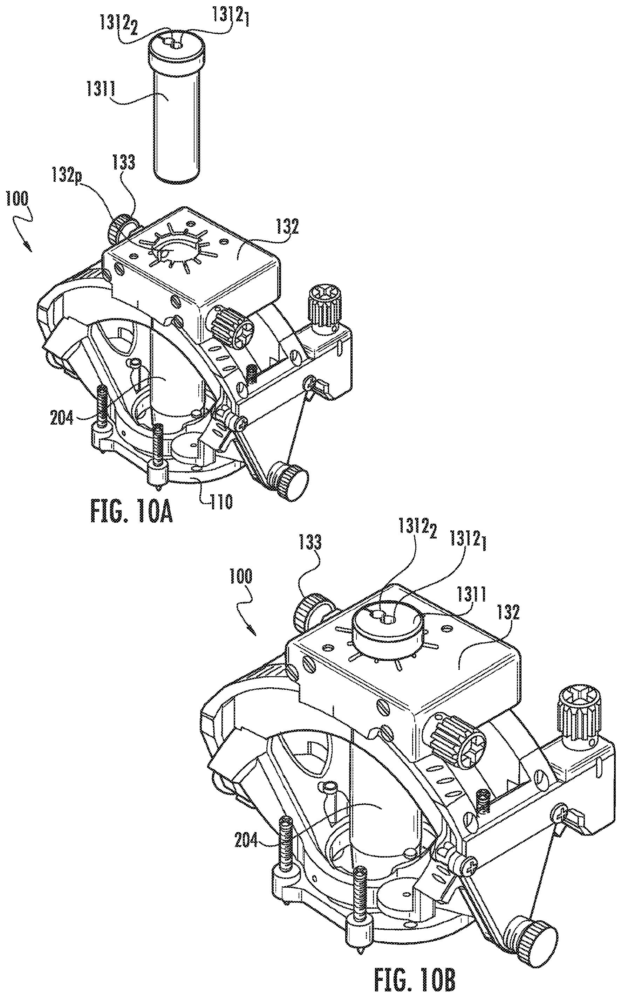

If a user has opted to create a smaller entry hole with a twistpoint drill then a twist point entry sequence can be followed as shown in **FIGS. 10A-10H****,** followed by use of a device guide **311** with the longitudinally extending open through channels **312 (****FIGS. 11A-11E****).** If a burr hole entry was performed, there is no requirement for use of a twist point entry guide **(1311,** **FIGS. 9A-9C****)** and a user can remove the fluid filled guide **211** and exchange it with the device guide **311 (****FIGS. 11A-11E****)** which is then used to insert the medical device **1000 (****FIGS. 11F****,** **11G).** The user can perform the trajectory selection steps with the fluid-filled array **211** for a burr hole procedure, to pick a safe path to insert a device through the brain. This can be carried out before swapping the fluid-filled guide **211** with a device guide **311.**

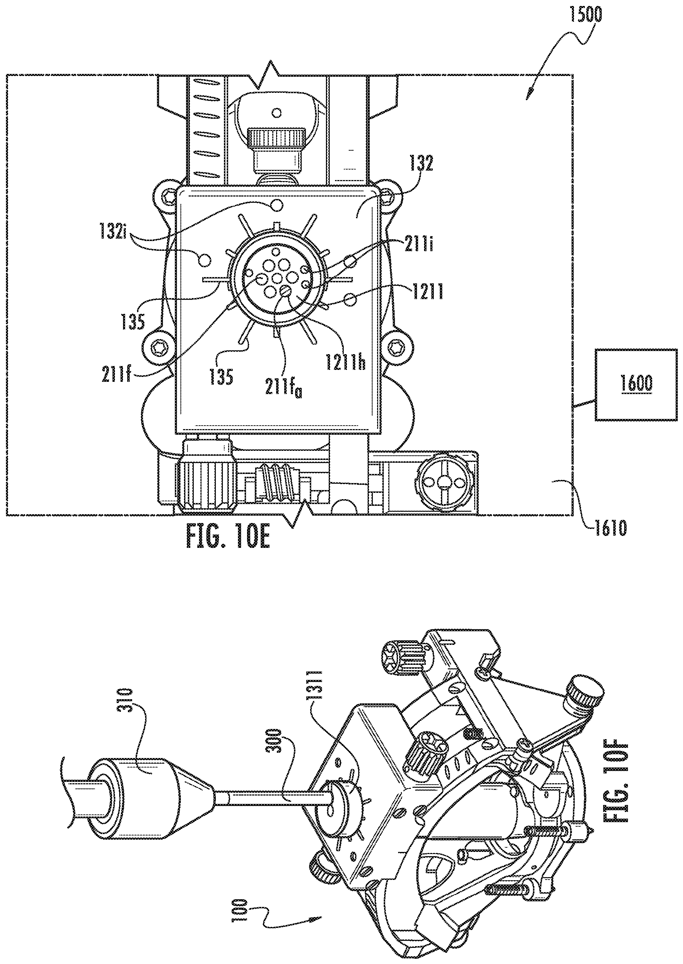

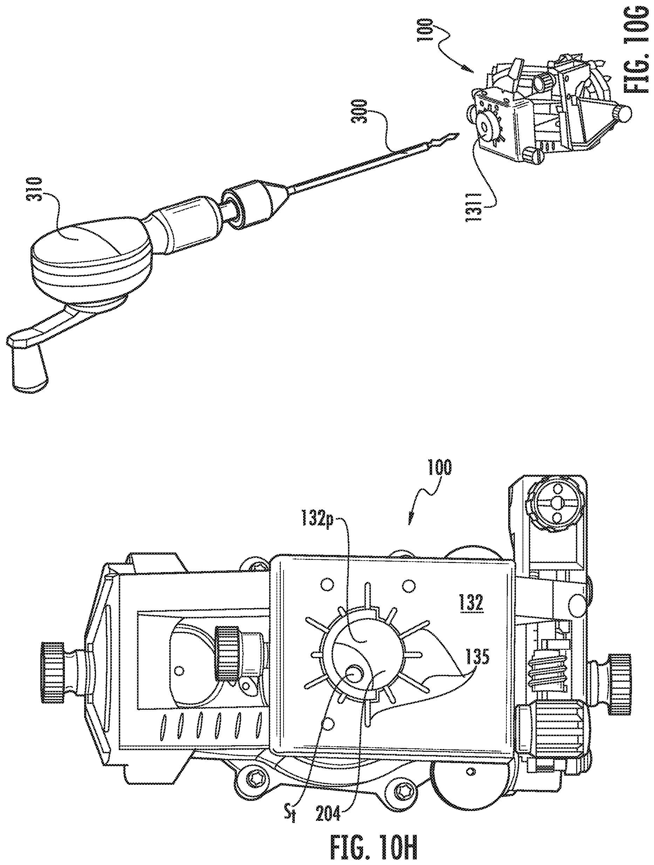

Referring to **FIGS. 9A-9C****,** a device guide **1311** can be inserted into the tower of the trajectory guide **100.** The device guide **1311** is sized and configured to hold a drill bit **300 (****FIGS. 10F****,** **10G)** for a twist drill **310 (****FIGS. 10F****,** **10G).** Typically, the inner diameters of the channels **1312** can be about 3.4 mm, about 4.5 mm, about 6.0 mm and about 9.0 mm. The center guide **1311c** may have a channel **1312** with a larger inner diameter. The rotatable combination guide **1311c** can be configured with the first channel **1312₁** having a larger diameter than the second channel **1312₂.** The larger size channel can be the center channel. The first and second channels **1312,** can, in some embodiments include a 3.4 mm inner diameter channel and a 4.5 mm inner diameter channel. Other device guides can be used with other configurations to support drill bits or larger sized therapeutic devices. A multi-lumen device guide **311** suitable for smaller size devices is shown in **FIGS. 11A-11E****.**

**FIG. 9A** illustrates a center guide **1311c** with a longitudinally extending guide channel **1312** centered side to side (laterally), i.e., centered with a longitudinally extending/axially extending center line. **FIG. 9B** discloses the device guide of the invention. **Fig. 9B** illustrates an offset device guide **1311o** as the device guide **1311.** The offset device guide **1311o** is a rotatable guide with a guide channel **1312** that is laterally offset from a longitudinally extending/axially extending center line. **FIG. 9C** illustrates a combination guide **1311m,** that can include first and second guide channels **1312,** that are longitudinally extending and adjacent each other, one of which can be centered and one of which can be laterally offset from center.

**FIG. 10A** is a top, side perspective views of the device guide **1311** (shown by way of example with device guide **1311c)** aligned with the trajectory frame **100** according to embodiments of the present invention. **FIG. 10B** illustrates the device guide **1311** assembled to the trajectory frame **100** and rotationally locked into position using fixation member **133.**

Referring to **FIGS. 10A-10D****,** of a surgeon decides that an offset hole should be created, then the offset guide **1311o (****FIG. 9B****)** or the combination guide **1311m** (as shown) can be rotated and locked to a desired offset position to create the entry hole St **(****FIG. 10H****).** If no offset is needed, the center guide **1311c** or the combination guide **1311m** can be used.

As shown in **FIG. 10E****,** a surgical (navigation) system **1400** can include an image processing circuit **1600** in communication with a display **1610.** The image processing circuit **1600** can generate an image **1500** that aligns the trajectory frame **100** and device guide **1311** and visually illustrates a desired fluid filled channel **211f that** is the one that should be used for alignment **211fa.** This alignment channel **2111fa** can be highlighted, colored, darkened or otherwise visually distinguished from other fluid filled channels **211f.** Thus, the image **1500** can include a virtual representation **1211** of the guide array **211** with the selected channel **211f** previously identified for the selected trajectory visually distinguished. The guide **1311** can be rotated to align the guide channel **1312** with the visually distinguished channel **211fa.** The visually distinguished channel **211fa** can be displayed in a first color different from one or other colors of other fluid filled lumens, or shown in black or white while the other fluid channels **211f** are displayed in a different color or in black when the alignment channel **211fa** is shown in white or in white when the alignment channel **211fa** is shown in black.

The surgeon can use the image **1500,** typically an MRI image or a visualization, to display one or more fluid filled guide channel(s) **211f** (virtually as the actual guide **211** is not on the trajectory frame **100** during this action) and directional channels **211i** along with the alignment indicia **132i** on the platform **321** to determine which direction to rotate and by how much. When the guide **1311** is rotated to an orientation that aligns one of the channels **1312** with a pre-selected trajectory associated with one of the one or more fluid filled lumens **211f** of the fluid-filled guide **211,** the device guide **1311** can be locked into position using fixation member **133.**

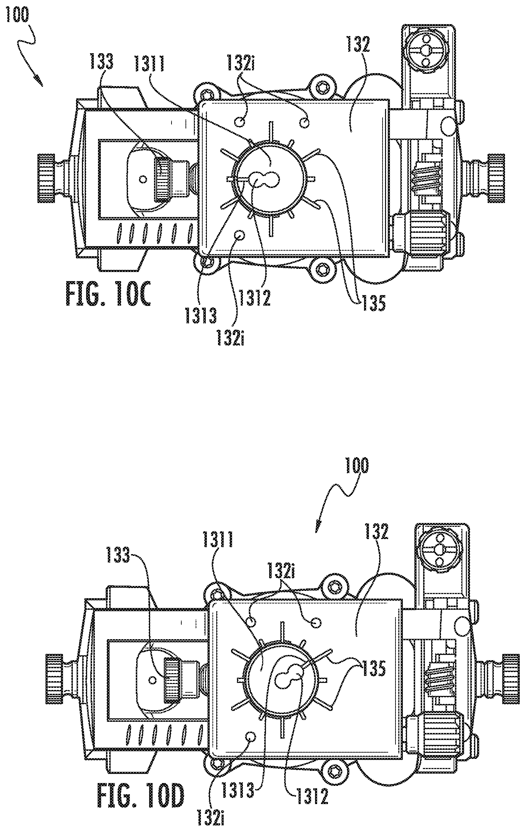

Referring to **FIGS. 10C** and **10D****,** the platform **132** can include straight outwardly extending lines **135** on an upper surface thereof that are circumferentially spaced apart and extend radially out from the port **132p.** At least some (i.e., the longer lines) or each of the lines **135** can extend radially outward from a position of a respective fluid filled guide channel **211f** that has a fixed rotational orientation in the platform **132.** The guide device **1311** can have a radially extending straight line **1313** that is aligned with a laterally extending centerline of the channel **1312.** This line **1313** can align with one of the lines **135** to help identify a fluid filled guide channel **211f.** This line **1313** can align with one of the lines **135** to help position the device guide channel **1312** over the trajectory the user has previously selected from the fluid-filled array channel **211f.**

**FIGS. 10F** and **10G** show a drill **310** and drill bit **300** cooperating with the device guide **1311** and trajectory frame **100.** The drill bit **300** can be inserted into the guide channel **1312** of the device guide **1311** and the drill **310** can be actuated to form the entry hole **St** as shown in **FIG. 10H****.**

**FIGS. 11A-11C** illustrate that a multi-lumen guide **311** with a plurality of spaced apart open through lumens/channels **312** can be coupled to the trajectory frame **100.** This guide **311** can be used to place/insert therapeutic devices **1000** into the patient to the target site. The multi-lumen guide **311** can have the same number of channels **312** as the fluid filled guide **211** and these channels **312** can be in the same position. As shown, there are seven channels **312.**

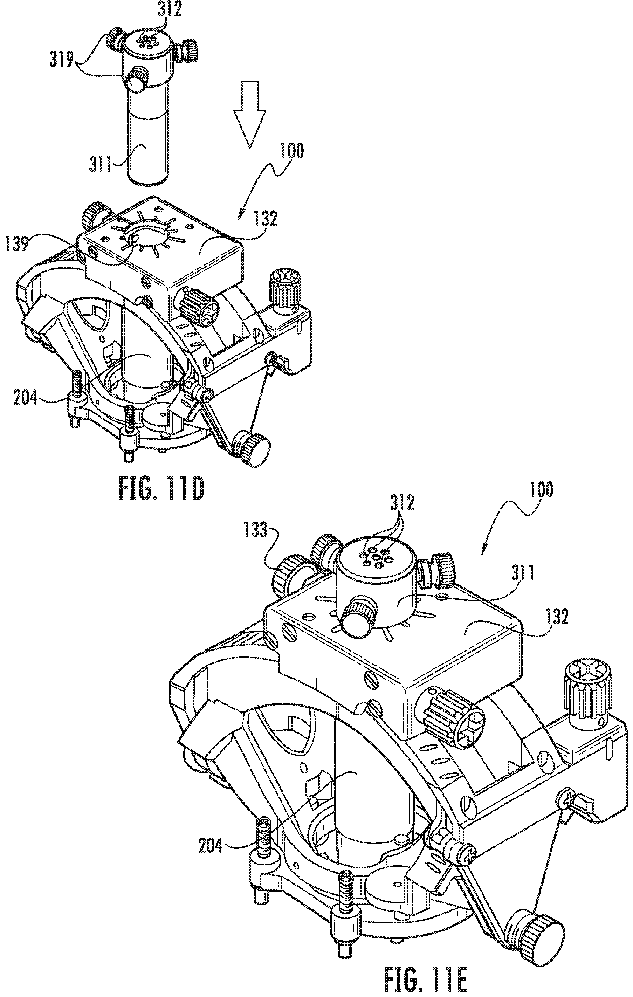

The guide **311** can have an external alignment feature **313** that cooperates with feature **139** in the platform **132** so that it the channels **311** have the same orientation as the channels **211f** when attached to the platform. The alignment feature **313** can have the same shape as that of **213** of the guide **211** with the fluid filled lumen(s) **211f.** As shown, the alignment feature **313** is a projecting ledge while the mating feature **139 (****FIG. 11D****)** is an enlarged perimeter segment of the port **132.** However, other affirmative alignment configurations may be used.

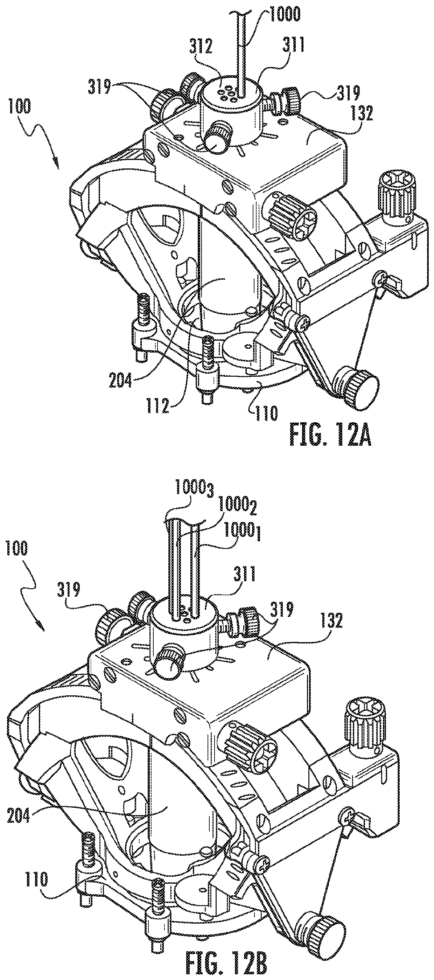

Still referring to **FIGS.11A-11C****,** the device guide **311** can have a plurality of circumferentially spaced apart fixation members **319** that reside on a top portion of the device guide and reside above the platform **132,** when in position **(****FIG. 11E****).** The fixation members **319** can lock against a therapeutic medical device **1000** to fix the device in a longitudinal position, i.e., so that the device **1000** cannot move up or down **(****FIGS. 12A****,** **12B).**

Referring to **FIG. 11C****,** the device guide **311** with the open through lumens **312** is shown adjacent the fluid filled lumen guide **211.** As shown, the top **311t** of the device guide **311** can have a planar surface and a perimeter region **320** that is free of any channels (unlike the alignment channels **211i** in the fluid-filled guide **211).** That is, all channels **312** can be open through channels and can have the same diameter.

**FIG. 11D** illustrates the multi-lumen guide **311** oriented to align with the alignment feature **139.** **FIG. 11E** illustrates the multi-lumen guide **311** releasably coupled to the trajectory frame **100** with a lower portion thereof in the tubular support **204** and locked via fixation member **133.**

**FIG. 11F** illustrates an example therapeutic device **1000** aligned with one of the open channels **312** of the multi-lumen guide **311.** The therapeutic device **1110** can have an adjustable depth stop member **1110.** The insertion depth calculated earlier can be marked on the device **1000,** measuring from the distal end **1000d.** The depth stop **1110** can be attached so that a bottom of the depth stop is aligned with the depth mark **1100m.** The device 1000 can then be inserted into the desired guide path of a selected open channel **312** until the depth stop bottoms out on top **311t** of the guide **311.**

**FIGS. 12A** and **12B** illustrate fixation members **319** that can be used to secure the therapeutic device **1000** in place. A single therapeutic device **1000** can be inserted through the multi-lumen guide **311** as shown in **FIG. 12A****.** Multiple therapeutic devices **1000₁, 1000₂, 1000₃** can be inserted through different channels **312** of the multi-lumen device **311** to be concurrently in position as shown in **FIG. 12B****.**

In some embodiments, the entire procedure can be carried out inside an MRI scanner room of an MRI suite and a different set of trajectory alignment and selection tools can be used from that shown in **FIGS. 5A-5H****.**

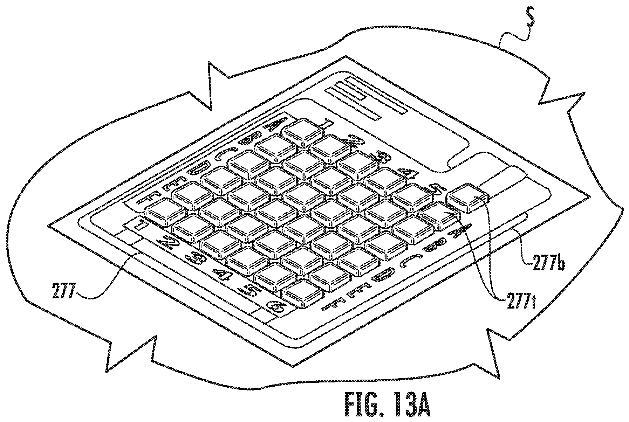

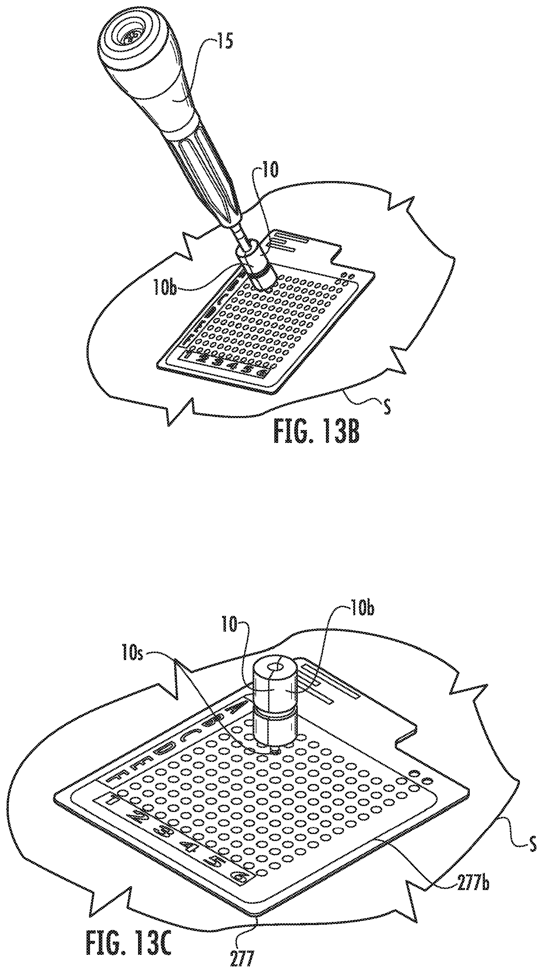

Referring now to **FIG. 13A****,** a fluid-filled grid **277** can be placed on a subject, i.e., on the head and/or skull **S.** An MRI scan can be performed encompassing the volume of the grid **277** and the intrabody target region of interest. The surgeon can choose an entry point through the grid **277** using automated or semi-automated trajectory selection/identification navigation systems. *See,* U.S. Patent Nos. 8,195,272 and 8,315,689, As discussed above, a surgeon can elect Option 1 (twist point entry via a twist drill) or Option 2 (larger burr hole) options for creating access for the surgical procedure. For the twist point entry, the centering screw guide **10 (****FIGS. 2D****,** **2E)** that uses a bone screw to directly anchor over the selected entry point can be used.

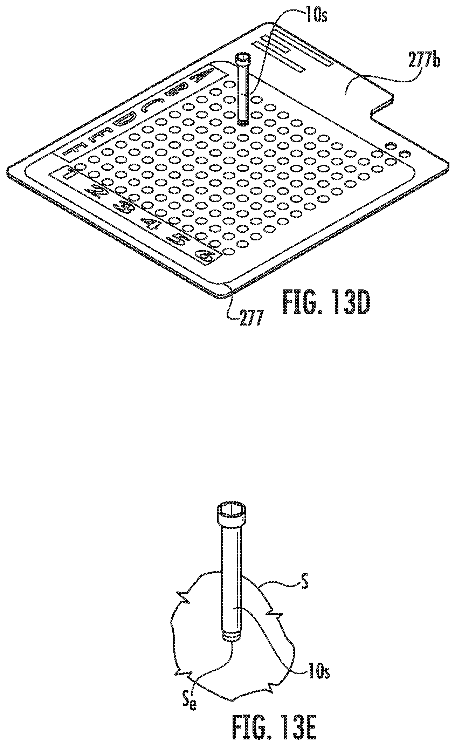

Referring to **FIGS. 13A-13C****,** the fluid-filled top **277t** of the grid **277** can be peeled off, leaving the base grid exposed **277b.** The centering screw guide **10** can be coupled to the skull using a screw driver **15,** directly onto the selected entry point through the grid base **277b.** Referring to **FIG. 13D****,** the primary body **10b** of the centering guide **10** can be removed leaving the centering guide bone screw **10s** in place through the grid base **277b.** As shown in **FIG. 13E****,** the grid **277** is removed (peeled off the skull), leaving the centering screw **10s** in place attached to the skull. The centering guide body **10b** can be reattached to the screw **10s** so that the centering tool **18 (****FIG. 2D****,** **2E)** can be inserted directly over it. As discussed above with respect to **FIGS. 2D** and **2E****,** the centering tool **18 (****FIG. 2D****)** can be attached to the centering screw guide (concentrically over and onto the guide **10).**

If Option 2 is elected, the surgeon can make a divot on the patient skull through the selected entry point on the marking grid using a marking too. Then, the same protocol as discussed with respect to **FIGS. 2F** and **2G** can be used to create a relatively large burr hole in the patient's skull.

As discussed above with respect to **FIGS. 3A-3C****,** the base **110** of the trajectory frame **100** can be centered over the centering tool **18** and coupled to the patient's skull through the scalp. Once the scalp mount base **110** is secured, the centering tool **18** and guide **10,** if used, are removed. As discussed above with respect to **FIGS. 4A-4E****,** the upper portion **100u** of the trajectory frame **100** can be attached to the base **110.**

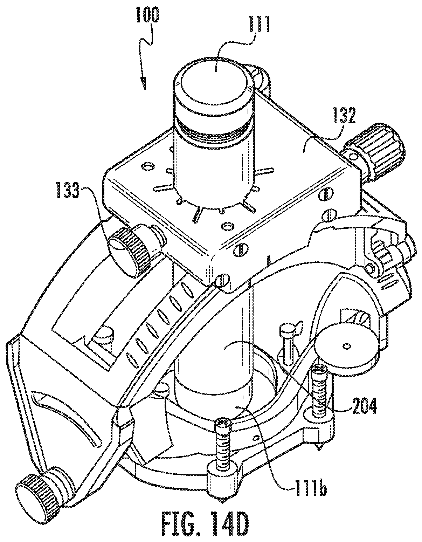

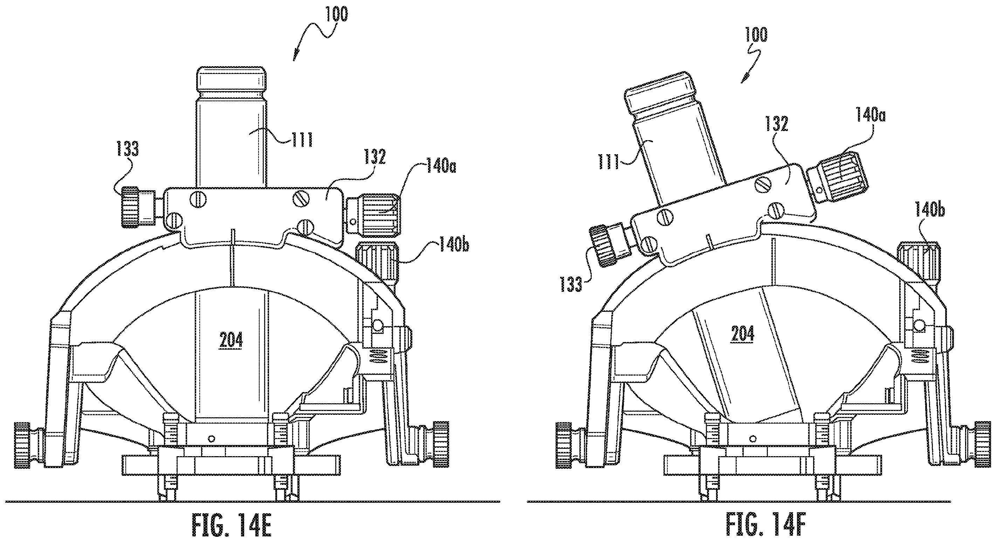

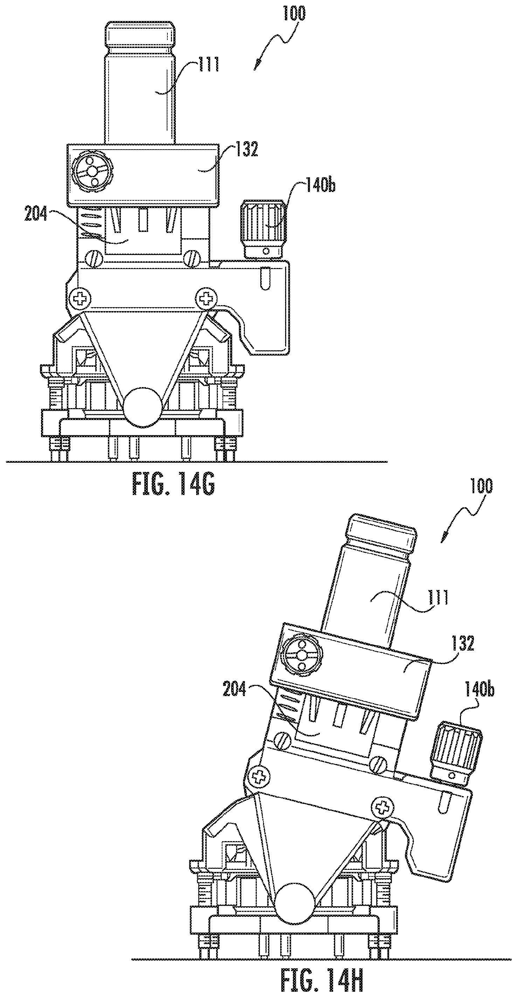

Referring now to **FIGS. 14A-14D****,** a targeting cannula **111** can be coupled to the trajectory frame **100** using the fixation thrum screw 133. The targeting cannula **111** has a fluid filled lumen (fluid column) **111f** and a cap **111c.** The targeting cannula **111** has a tubular body that has the inner lumen with a closed bottom end and a larger diameter reservoir **111r** above the fluid column **111f.** The bottom **111b** can extend out of the bottom of the tower or tubular member **204** when in position as shown in **FIGS. 14C** and **14D****.** An MRI scanner can scan the image volume with the intrabody target region of interest and the targeting cannula **111** and a surgical navigation system can electronically calculate positional adjustments (i.e., knob rotations for pitch and roll adjustment) to align the trajectory of the tubular member/tower **204** to the desired trajectory. **FIGS. 14E** and **14F** illustrate example pitch adjustment using pitch actuator **140a.** **FIGS. 14G** and **14H** illustrate example roll adjustment using roll actuator **140b.**

**FIGs. 14B** and **14C** illustrate that the pitch actuator **140a'** can be parallel to the tubular member **204** and/or device guide **311** or fluid filled guide **211** and can reside above the platform **132** on a corner of the platform **132** as shown. Thus, the roll and pitch actuators **140b, 140a** can be parallel to each other and extend in an upright direction.

After the trajectory adjustments to the tower **204** of the trajectory frame **100,** either via the navigation stylus **5 (****FIG. 5C****)** discussed above or a targeting cannula **111,** a multi-lumen fluid filled guide array **211** can be used to determine a trajectory selection channel of a guide **311** as discussed above **(****FIGS. 6A-6C****,** **FIGS. 7A-7E** and **8).** Once the guide array **211** is inserted, an MRI scan that encompasses the volume of the trajectory frame **100** and the intrabody region of interest/target can be performed and pitch and roll adjustments made. The navigation stylus **5** can be used for the CT imaging modality while an MR visible targeting cannula **111** and/or fluid-filled array **211** can be used for an MRI only workflow.

Thus, in some embodiments, after a trajectory is set, the targeting cannula **111** can be removed from the tower **204,** and a fluid-filled guide array **211** can used to pick a path to the target. There are a plurality (shown as seven) possible device paths included in the guide array **211,** to give the surgeon multiple options for selecting the safest path to reach the desired target. Also, these additional paths act as a way to counter-act any mounting errors that may have occurred.

The fluid filled guide channels **211f (****FIGS. 6D****, 6E)** of the guide array **211** will be bright parallel lines on an MRI image obtained by an MRI scan or scans. The surgeon can select the lumen position in the array that most closely matches the desired insertion path/trajectory.

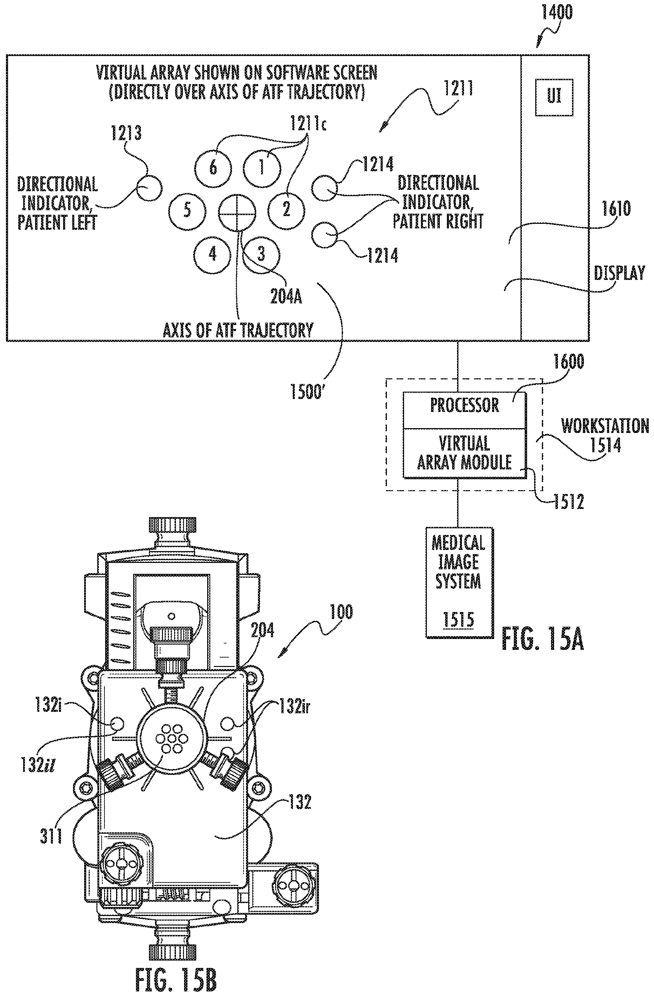

Alternatively, instead of (or even in combination with) the physical guide array **211,** a virtual a multi-lumen fluid filled guide array **1211 (****FIG. 15A****)** can be generated programmatically, and digitally overlaid onto an MRI image comprising the trajectory frame **100.** Thus, the virtual array **1211** can be used to select a corresponding lumen position in a multi-lumen guide **311 (****FIGS. 8** and **11A****-11C)** as will be discussed below.

As shown in **FIG. 15A****,** a surgical system **1400** can include an image processing circuit **1600** in communication with the display **1610.** The image processing circuit **1600** can generate an image **1500'** that aligns a virtual guide array **1211** and the axis of the tubular member **204** held by the trajectory guide assembly **100.**

Referring to **FIGS. 15A** and **15B****,** once a "final" or "set" trajectory alignment has been made, a user interface can prompt a user to select a desired trajectory. The surgical navigation system **1400** can include a display **1610** in communication with a processor **1160** with a virtual array module **1512** that can programmatically generate and automatically overlay a virtual guide array **1211** onto an image **1500',** centered axially (indicated by cross-hair center) on longitudinal axis **204A** of the tower **204.** The system **1400** can be in communication with or at least partially onboard a medical imaging system **1515** such as an MRI imaging system (and/or optionally a CT imaging system). The processor **1600** and display **1610** can be provided as components of a workstation **1514.**

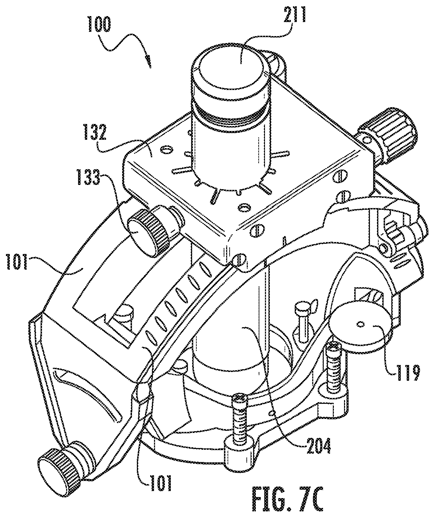

The system **1400** can automatically orient the virtual array **1211** optionally based on orientation of circumferentially spaced apart fiducial markers **119 (****FIGS. 3A****,** **7C)** on the base **110** of the trajectory frame **100.** The virtual array **1211** matches the virtual channels **1211c** with the physical channels of the multi-lumen guide **311 (****FIGS. 8****,** **11A-11C****,** **15B****)** and orientation indicia **132i** on platform **132 (****FIGS. 7E****,** **15B****).** The virtual array **1211** can include a plurality of circumferentially and radially spaced apart virtual channels **1211c,** shown as seven channels numbered as channels 1-7, with a center channel (channel 7) surrounded by a concentric set of six equally circumferentially spaced apart channels (numbered as channels 1-6). The virtual array **1211** can also include virtual orientation indicia including a virtual patient left directional indicator **1213** and a virtual directional patient right directional indicator **1214** (shown as a pair of right side markings). The virtual patient left marking **1213** can be aligned and provided to visually match or correspond to the patient left marking **132il** (on the platform **132)** and the virtual patient right marking **1214** can visually correspond to the patient right marking **132ir** (on the platform **132)** with the virtual markings positioned aligned but radially spaced apart and closer to the tubular member **204** and/or center of the platform **132** than the physical markings **132i.** The surgeon then selects which path (virtual channel **1211c)** most closely matches the desired trajectory.

If the user has opted to create a smaller entry hole with a twist point drill, then the protocol discussed above with respect to **FIGS. 8****,** **9A-9C****,** **10A-10H** can be performed followed by the use of the multi-lumen guide **311** discussed with respect to **FIGS. 11A-11C****.** If a burr hole entry was performed, then the multi-lumen guide **311** can be attached to the trajectory frame **100** without requiring the twist entry tools and steps.

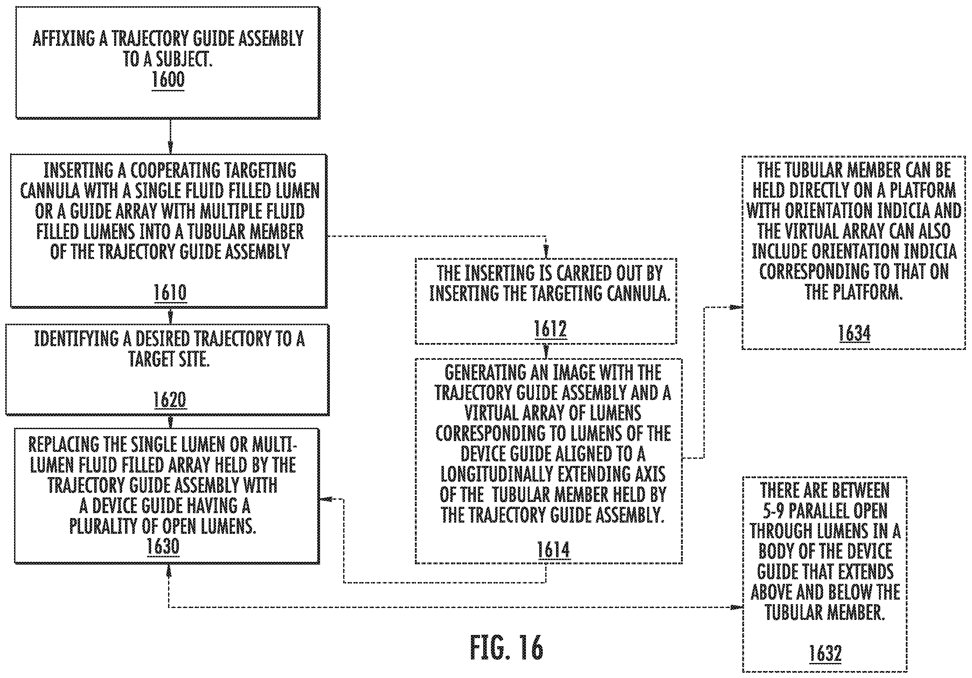

**FIG. 16** is a flow chart of example actions that can be used for surgical navigation for a therapeutic treatment according to embodiments of the present invention. A trajectory guide assembly can be affixed to a subject (block **1600).** A cooperating device comprising either a targeting cannula with a single fluid filled lumen or a guide array with multiple fluid filled lumens can be inserted into a tubular member (tower) of the trajectory guide assembly (block **1610).** A desired trajectory can be identified using the inserted device (block **1620).** The device can be replaced with a device guide having a plurality of open lumens (block **1630).**

The insertion can be carried out by inserting the targeting cannula (block **1612).** The method can include generating an image with the trajectory guide assembly and a virtual array of lumens corresponding to the lumens of the device guide aligned to a longitudinally extending axis of the tubular member held by the trajectory guide assembly (block **1614).** There can be between 5-9 parallel and open through lumens in a body of the device guide that extends above and below the tubular member (block **1632).** The tubular member can be held directly on a platform with orientation indicia and the virtual array can also include orientation indicia corresponding to that on the platform (block **1634).**

The surgical navigation system **1500 (****FIG. 15A****)** can take the form of an entirely software embodiment or an embodiment combining software and hardware aspects, all generally referred to herein as a "circuit" or "module". Furthermore, the present invention may take the form of a computer program product on a computer-usable storage medium having computer-usable program code embodied in the medium. Any suitable computer readable medium may be utilized including hard disks, CD-ROMs, optical storage devices, a transmission media such as those supporting the Internet or an intranet, or magnetic storage devices. Some circuits, modules or routines may be written in assembly language or even micro-code to enhance performance and/or memory usage. It will be further appreciated that the functionality of any or all of the program modules may also be implemented using discrete hardware components, one or more application specific integrated circuits (ASICs), or a programmed digital signal processor or microcontroller. Embodiments of the present invention are not limited to a particular programming language.

Computer program code for carrying out operations of data processing systems, method steps or actions, modules or circuits (or portions thereof) discussed herein may be written in a high-level programming language, such as Python, Java, AJAX (Asynchronous JavaScript), C, and/or C++, for development convenience. In addition, computer program code for carrying out operations of exemplary embodiments may also be written in other programming languages, such as, but not limited to, interpreted languages. Some modules or routines may be written in assembly language or even micro-code to enhance performance and/or memory usage. However, embodiments are not limited to a particular programming language. As noted above, the functionality of any or all of the program modules may also be implemented using discrete hardware components, one or more application specific integrated circuits (ASICs), or a programmed digital signal processor or microcontroller. The program code may execute entirely on one (e.g., a workstation computer), partly on one computer, as a stand-alone software package, partly on the workstation's computer or Scanner's computer and partly on another computer, local and/or remote or entirely on the other local or remote computer. In the latter scenario, the other local or remote computer may be connected to the user's computer through a local area network (LAN) or a wide area network (WAN), or the connection may be made to an external computer (for example, through the Internet using an Internet Service Provider).

The present invention is described in part with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function/act specified in the flowchart and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing some or all of the functions/acts specified in the flowchart and/or block diagram block or blocks.

The flowcharts and block diagrams of certain of the figures herein illustrate exemplary architecture, functionality, and operation of possible implementations of embodiments of the present invention. In this regard, each block in the flow charts or block diagrams represents a module, segment, or portion of code, which comprises one or more executable instructions for implementing the specified logical function(s). It should also be noted that in some alternative implementations, the functions noted in the blocks may occur out of the order noted in the figures. For example, two blocks shown in succession may in fact be executed substantially concurrently or the blocks may sometimes be executed in the reverse order or two or more blocks may be combined, depending upon the functionality involved.

As illustrated in **FIG.17****,** embodiments of the invention may be configured as a data processing system **2000,** which can be used to carry out or direct operations of the surgical navigation system **1400,** and can include a processor **1600,** a memory **2336** and input/output circuits **2346.** The data processing system may be incorporated in, for example, one or more of a personal computer, workstation **1514 (****FIG. 15A****),** server(s) or the like. The system **2000** can reside on one machine or be distributed over a plurality of machines and/or be a cloud based system. The processor **1600** communicates with the memory **2336** via an address/data bus **2348** and communicates with the input/output circuits **2346** via an address/data bus **2349.** The input/output circuits **2346** can be used to transfer information between the memory (memory and/or storage media) **2336** and another computer system or a network using, for example, an Internet protocol (IP) connection. These components may be conventional components such as those used in many conventional data processing systems, which may be configured to operate as described herein.

In particular, the processor **1510** can be commercially available or custom microprocessor, microcontroller, digital signal processor or the like. The memory **2336** may include any memory devices and/or storage media containing the software and data used to implement the functionality circuits or modules used in accordance with embodiments of the present invention. The memory **2336** can include, but is not limited to, the following types of devices: ROM, PROM, EPROM, EEPROM, flash memory, SRAM, DRAM and magnetic disk. In some embodiments of the present invention, the memory **336** may be a content addressable memory (CAM).

As further illustrated in **FIG. 17****,** the memory (and/or storage media) **2336** may include several categories of software and data used in the data processing system: an operating system **2352;** application programs **2354;** input/output device drivers **2358;** and data **2356.** As will be appreciated by those of skill in the art, the operating system **2352** may be any operating system suitable for use with a data processing system, such as IBM^{®}, AIX^{®} or zOS^{®} operating systems or Microsoft^{®} Windows2000 or WindowsXP operating systems, Windows Visa, Windows7, Windows CE or other Windows versions from Microsoft Corporation, Redmond, WA, Palm OS, Symbian OS, Cisco IOS, VxWorks, Unix or Linux^{™}, Mac OS from Apple Computer, LabView, or proprietary operating systems. IBM, AIX and zOS are trademarks of International Business Machines Corporation in the United States, other countries, or both while Linux is a trademark of Linus Torvalds in the United States, other countries, or both. Microsoft and Windows are trademarks of Microsoft Corporation in the United States, other countries, or both. The input/output device drivers **2358** typically include software routines accessed through the operating system **2352** by the application programs **2354** to communicate with devices such as the input/output circuits **2346** and certain memory **2336** components. The application programs **2354** are illustrative of the programs that implement the various features of the circuits and modules according to some embodiments of the present invention. Finally, the data **2356** represents the static and dynamic data used by the application programs **2354** the operating system **2352** the input/output device drivers **2358** and other software programs that may reside in the memory **2336.**

The data **2356** may include (near real time or archived or stored) digital image data sets **2326** that provide image data including image volumes encompassing the trajectory frame and intrabody target (typically also comprising DICOM data to correlate the image data to respective patients). The data **2356** may include defined trajectory frame orientation features such as fiducial features and positions for defining an orientation of the trajectory frame **100** in image space and/or to patient right, patient front and patient left.

As further illustrated in **FIG. 17****,** according to some embodiments of the present invention application programs **2354** include a Device Guide Module for a device guide with a plurality of parallel open lumens **2324** and an optional Virtual Array Module **1512.** The application program **2354** may be located in a local server (or processor) and/or database or a remote server (or processor) and/or database, or combinations of local and remote databases and/or servers.

While the present invention is illustrated with reference to the application programs **2354,** and Modules **2324, 1512** in **FIG. 17****,** as will be appreciated by those of skill in the art, other configurations fall within the scope of the present invention. For example, rather than being application programs **2354** these circuits and modules may also be incorporated into the operating system **2352** or other such logical division of the data processing system. Furthermore, while the application programs **2354** are illustrated in a single data processing system, as will be appreciated by those of skill in the art, such functionality may be distributed across one or more data processing systems in, for example, the type of client/server arrangement described above. Thus, the present invention should not be construed as limited to the configurations illustrated in **FIG. 17** but may be provided by other arrangements and/or divisions of functions between data processing systems. For example, although **FIG. 17** is illustrated as having various circuits and modules, one or more of these circuits or modules may be combined or separated without departing from the scope of the present invention.

In particular embodiments, the system **1400** can include or be in communication with a PACS (picture archiving and communication) system. The system **1500** can include, for example, at least one server and/or at least one (clinical) client (e.g., workstation).

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the scope of this invention. Accordingly, all such modifications are limited by the scope of this invention as defined in the claims. The invention is defined by the following claims, with equivalents of the claims to be included therein.

## Claims

1. A surgical navigation system (1400), comprising:
a trajectory guide assembly (100) comprising:
a base (110) having a patient access aperture formed therein, wherein the base (110) is configured to be secured to the body of a patient;
a yoke (120) movably mounted to the base (110) and rotatable about a first axis; and
a platform (132) with an open port (132p) that is movably mounted to the yoke (120) and rotatable about a second axis;
a trajectory selection guide member (111, 211) comprising at least one longitudinally extending fluid filled channel of one or more contrast agents releasably attachable to the platform (132); and
a device guide (311,1311) sized and configured to hold a drill bit (300), wherein the device guide is releasably attachable to the platform, further wherein the device guide (311,1311) is a rotatable offset guide (1311o) comprising only one longitudinally extending channel (1312) that is offset from an axially extending centerline of the device guide (311, 1311), and wherein the trajectory selection guide member (211) and the device guide (311,1311) are serially interchangeably held by the platform (132) and each have a length sufficient to extend through the port (132p) of the platform (132) with a bottom portion thereof residing a distance below the platform (132).

2. The system of Claim 1, further comprising an image processing circuit (1600) configured to generate and display a virtual trajectory selection guide member (1211) configured as a virtual multi-lumen guide array and aligned with an image (1500) of the trajectory guide assembly (100), wherein the virtual multi-lumen guide array comprises a plurality of radially and/or circumferentially spaced apart virtual channels spaced apart about a virtual center channel, and wherein the virtual center channel is aligned with a center of the open port (132p) of the platform (132).

3. The system of Claim 1, wherein the platform (132) comprises visual orientation indicia (132i) on an upper surface (132u) thereof that includes a patient right directional indicator (D_{R}), a patient left directional indicator (D_{L}) and a forward directional indicator (D_{F}), wherein the patient right directional indicator (D_{R}), the patient left directional indicator (D_{L}) and the forward directional indicator (D_{F}) are provided as respective markings on the upper surface (132u).

4. The system of Claim 1, wherein the trajectory selection guide member (111,211) comprises an upper surface with visual orientation indicia (211i) including a patient right directional indicator (D_{R}), a patient left directional indicator (D_{L}) and a forward directional indicator (D_{F}).

5. The system of Claim 1, wherein the trajectory selection guide member (111, 211) comprises a cap (211c) sealably attached to and enclosing a primary body (211b), wherein the cap (211c) resides above a liquid reservoir (211fₜ), and wherein the liquid reservoir (211fₜ) has a width that is larger than a width of the at least one longitudinally extending fluid filled lumen and merges into the at least one longitudinally extending fluid filled channel.

6. The system of Claim 1, wherein the platform (132) is rectangular, wherein the system further comprises a tubular support member (204) held by the platform (132) and extending under the open port (132p), and wherein the open port (132p) of the platform (132) comprises a perimeter with an alignment feature (139) that circumferentially extends about a sub-set of the perimeter and that slidably receives a matable alignment feature (213) on the device guide (311, 1311).

7. The system of Claim 1, wherein the device guide (311, 1311) is a first device guide, and wherein the system further comprises a second device guide that is also releasably and interchangeably extended through the port of the platform and is directly secured to the platform, wherein the second device guide is
a center guide (1311c) with a longitudinally extending channel (1312) that is centered with an axially extending centerline of the center guide (1311c).

8. The system of Claim 1, wherein the trajectory selection guide member (111, 211) is a multi-lumen guide array (211) that comprises a plurality of radially and/or circumferentially spaced apart fluid filled lumens (211f) spaced apart about a center fluid filled lumen.

9. The system of Claim 1, wherein the trajectory selection guide member (111, 211) is a multi-lumen guide array (211) with a plurality of radially and/or circumferentially spaced apart fluid filled lumens (211f), and wherein the fluid filled channels (211f) terminate at a top end under a cap (211c), and wherein the device guide (311, 1311) has a top end that is at the same height as the top end of the fluid filled channels.

10. The system of Claim 1, wherein the trajectory guide assembly (100) further comprises a pair of arcuate laterally spaced apart arms (101) that hold the platform (132) therebetween and above the base(110) and only two actuators (140a, 140b) for pitch and roll, and wherein the trajectory guide assembly (100) is devoid of x-y direction actuators.

11. The system of Claim 10, wherein the platform (132) is slidably supported by the arms (101) to thereby allow the platform (132) to slidably travel forward and rearward over a curvilinear path defined by the arms (101).

12. The system of Claim 9, wherein the plurality of fluid filled channels (211f) have a common length.

13. The system of Claim 1, wherein the trajectory selection guide member (111, 211) is a multi-lumen guide array (211) with a plurality of radially and/or circumferentially spaced apart fluid filled lumens (211f) spaced apart about a center fluid filled lumen, and wherein the plurality of fluid filled channels of the multi-lumen guide array is seven.

14. The system of Claim 1, wherein the trajectory selection guide member (111, 211) is a multi-lumen guide array (211) with a plurality of spaced apart longitudinally extending fluid filled lumens (211f), wherein the plurality of longitudinally extending fluid filled channels comprise a center channel with adjacent channels residing spaced apart about the center channel, wherein the multi-lumen guide array (211) comprises orientation indicia (2111) corresponding to patient directions of right, left and forward, and wherein the platform (132) has corresponding orientation indicia (132i).

15. The system of Claim 1, wherein the platform (132) comprises an upper surface (132u) with orientation indicia (132i) and a plurality of straight radially outwardly extending lines (135) on the upper surface thereof that are circumferentially spaced apart and extend radially out from the open port (132p).

## Patentansprüche

1. Chirurgisches Navigationssystem (1400), umfassend:
eine Trajektorienführungsanordnung (100), umfassend:
eine Basis (110) mit einer darin gebildeten Patientenzugangsöffnung, wobei die Basis (110) konfiguriert ist, um an dem Körper eines Patienten befestigt zu werden;
eine Gabel (120), die beweglich an der Basis (110) montiert und um eine erste Achse herum drehbar ist; und
eine Plattform (132) mit einem offenen Port (132p), der beweglich an der Gabel (120) montiert und um eine zweite Achse herum drehbar ist;
ein Trajektorienauswahlführungselement (111, 211), das mindestens einen sich längsgerichtet erstreckenden, mit Fluid von einem oder mehreren Kontrastmitteln gefüllten Kanal umfasst, der lösbar an der Plattform (132) anbringbar ist; und
eine Vorrichtungsführung (311, 1311), die bemessen und konfiguriert ist, um einen Bohrer (300) zu halten, wobei die Vorrichtungsführung lösbar an der Plattform anbringbar ist, wobei die Vorrichtungsführung (311, 1311) weiter eine drehbare Versatzführung (1311o) ist, die nur einen sich längsgerichtet erstreckenden Kanal (1312) umfasst, der von einer sich axial erstreckenden Mittellinie der Vorrichtungsführung (311, 1311) versetzt ist, und wobei das Trajektorienauswahlführungselement (211) und die Vorrichtungsführung (311, 1311) seriell austauschbar von der Plattform (132) gehalten werden und jeweils eine Länge aufweisen, die ausreicht, um sich durch den Port (132p) der Plattform (132) zu erstrecken, mit einem unteren Abschnitt, der einen Abstand unterhalb der Plattform (132) liegt.

2. System nach Anspruch 1, weiter umfassend eine Bildverarbeitungsschaltung (1600), die konfiguriert ist, um ein virtuelles Trajektorienauswahlführungselement (1211) zu erzeugen und anzuzeigen, das als ein virtuelles Multilumenführungsarray konfiguriert und mit einem Bild (1500) der Trajektorienführungsanordnung (100) ausgerichtet ist, wobei das virtuelle Multilumenführungsarray eine Vielzahl von radial und/oder umlaufend beabstandeten virtuellen Kanälen umfasst, die um einen virtuellen Mittelkanal herum beabstandet sind, und wobei der virtuelle Mittelkanal mit einer Mitte des offenen Ports (132p) der Plattform (132) ausgerichtet ist.

3. System nach Anspruch 1, wobei die Plattform (132) auf einer oberen Oberfläche (132u) davon visuelle Orientierungszeichen (132i) umfasst, die einen Richtungsindikator für die rechte Seite eines Patienten (D_{R}), einen Richtungsindikator für die linke Seite eines Patienten (D_{L}) und einen Richtungsindikator nach vorne (D_{F}) einschließt, wobei der Richtungsindikator für die rechte Seite des Patienten (D_{R}), der Richtungsindikator für die linke Seite des Patienten (D_{L}) und der Richtungsindikator nach vorne (D_{F}) als jeweilige Markierungen auf der oberen Oberfläche (132u) bereitgestellt sind.

4. System nach Anspruch 1, wobei das Trajektorienauswahlführungselement (111, 211) eine obere Oberfläche mit visuellen Orientierungszeichen (211i) umfasst, einschließlich eines Richtungsindikators für die rechte Seite des Patienten (D_{R}), eines Richtungsindikators für die linke Seite des Patienten (D_{L}) und eines Richtungsindikators nach vorne (D_{F}).

5. System nach Anspruch 1, wobei das Trajektorienauswahlführungselement (111, 211) eine Kappe (211c) umfasst, die abdichtbar an einem Primärkörper (211b) angebracht ist und diesen umschließt, wobei die Kappe (211c) über einem Flüssigkeitsreservoir (211fₜ) liegt und wobei das Flüssigkeitsreservoir (211fₜ) eine Breite aufweist, die größer ist als eine Breite des mindestens einen sich längsgerichtet erstreckenden, mit Fluid gefüllten Lumens, und in den mindestens einen sich längsgerichtet erstreckenden, mit Fluid gefüllten Kanal übergeht.

6. System nach Anspruch 1, wobei die Plattform (132) rechteckig ist, wobei das System weiter ein rohrförmiges Stützelement (204) umfasst, das von der Plattform (132) gehalten wird und sich unter den offenen Port (132p) erstreckt, und wobei der offene Port (132p) der Plattform (132) einen Umfang mit einem Ausrichtungsmerkmal (139) umfasst, das sich umlaufend um einen Teil des Umfangs herum erstreckt und ein einpassbares Ausrichtungsmerkmal (213) an der Vorrichtungsführung (311, 1311) verschiebbar aufnimmt.

7. System nach Anspruch 1, wobei die Vorrichtungsführung (311, 1311) eine erste Vorrichtungsführung ist und wobei das System weiter eine zweite Vorrichtungsführung umfasst, die ebenfalls lösbar und austauschbar durch den Port der Plattform verlängert ist und direkt an der Plattform befestigt ist, wobei die zweite Vorrichtungsführung
eine Mittelführung (1311c) mit einem sich längsgerichtet erstreckenden Kanal (1312) ist, der mit einer sich axial erstreckenden Mittellinie der Mittelführung (1311c) zentriert ist.

8. System nach Anspruch 1, wobei das Trajektorienauswahlführungselement (111, 211) ein Multilumenführungsarray (211) ist, das eine Vielzahl von radial und/oder umlaufend beabstandeten, mit Fluid gefüllter Lumen (211f) umfasst, die um ein mittleres, mit Fluid gefülltes Lumen herum beabstandet sind.

9. System nach Anspruch 1, wobei das Trajektorienauswahlführungselement (111, 211) ein Multilumenführungsarray (211) mit einer Vielzahl von radial und/oder umlaufend beabstandeten, mit Fluid gefüllter Lumen (211f) ist, und wobei die mit Fluid gefüllten Kanäle (211f) an einem oberen Ende unter einer Kappe (211c) enden, und wobei die Vorrichtungsführung (311, 1311) ein oberes Ende aufweist, das auf der gleichen Höhe wie das obere Ende der mit Fluid gefüllten Kanäle vorliegt.

10. System nach Anspruch 1, wobei die Trajektorienführungsanordnung (100) weiter ein Paar von bogenförmigen, seitlich beabstandeten Arme (101), die die Plattform (132) dazwischen und über der Basis (110) halten, und nur zwei Betätigungsvorrichtungen (140a, 140b) für Nick- und Rollbewegungen umfasst, und wobei die Trajektorienführungsanordnung (100) frei von Betätigungsvorrichtungen in xy-Richtung ist.

11. System nach Anspruch 10, wobei die Plattform (132) von den Armen (101) verschiebbar gestützt wird, um der Plattform (132) dadurch zu erlauben, sich verschiebbar über einen durch die Arme (101) definierten gekrümmten Pfad nach vorne und nach hinten zu bewegen.

12. System nach Anspruch 9, wobei die Vielzahl von mit Fluid gefüllten Kanälen (211f) eine gemeinsame Länge aufweisen.

13. System nach Anspruch 1, wobei das Trajektorienauswahlführungselement (111, 211) ein Multilumenführungsarray (211) mit einer Vielzahl von radial und/oder umlaufend beabstandeten, mit Fluid gefüllten Lumen (211f) ist, die um ein mittleres, mit Fluid gefülltes Lumen herum beabstandet sind, und wobei die Vielzahl von mit Fluid gefüllten Kanälen des Multilumenführungsarrays sieben beträgt.

14. System nach Anspruch 1, wobei das Trajektorienauswahlführungselement (111, 211) ein Multilumenführungsarray (211) mit einer Vielzahl von beabstandeten, sich längsgerichtet erstreckenden, mit Fluid gefüllten Lumen (211f) ist, wobei die Vielzahl von sich längsgerichtet erstreckenden, mit Fluid gefüllten Kanälen einen Mittelkanal mit benachbarten Kanälen umfasst, die um den Mittelkanal herum beabstandet liegen, wobei das Multilumenführungsarray (211) Orientierungszeichen (211i) umfasst, die den Patientenrichtungen rechts, links und nach vorne entsprechen, und wobei die Plattform (132) entsprechende Orientierungszeichen (132i) aufweist.

15. System nach Anspruch 1, wobei die Plattform (132) eine obere Oberfläche (132u) mit Orientierungszeichen (132i) und eine Vielzahl von geraden, sich radial nach außen erstreckenden Linien (135) auf der oberen Oberfläche davon umfasst, die umlaufend beabstandet sind und sich von dem offenen Port (132p) radial heraus erstrecken.

## Revendications

1. Système de navigation chirurgical (1400), comprenant :
un ensemble de guidage de trajectoire (100) comprenant :
une base (110) comportant une ouverture d'accès au patient formée dans celle-ci, dans lequel la base (110) est configurée pour être fixée au corps d'un patient ;
un joug (120) monté de manière mobile sur la base (110) et pouvant tourner autour d'un premier axe ; et
une plateforme (132) avec un orifice ouvert (132p) qui est montée de manière mobile sur le joug (120) et rotative autour d'un deuxième axe ;
un élément de guidage de sélection de trajectoire (111, 211) comprenant au moins un canal rempli de fluide s'étendant longitudinalement et contenant un ou plusieurs agents de contraste pouvant être fixés de manière amovible à la plateforme (132) ; et
un guide de dispositif (311,1311) dimensionné et configuré pour contenir un foret (300), dans lequel le guide de dispositif est amovible et peut être fixé à la plateforme, en outre dans lequel le guide de dispositif (311,1311) est un guide décalé rotatif (1311o) ne comprenant qu'un seul canal longitudinal (1312) décalé par rapport à l'axe central axial du guide de dispositif (311, 1311), et dans lequel l'élément de guide de sélection de trajectoire (211) et le guide de dispositif (311,1311) sont maintenus en série de manière interchangeable par la plateforme (132) et chacun présente une longueur suffisante pour s'étendre à travers l'orifice (132p) de la plateforme (132) avec une partie inférieure de celui-ci résidant à une distance en dessous de la plateforme (132).

2. Système selon la revendication 1, comprenant en outre un circuit de traitement d'image (1600) configuré pour générer et afficher un guide de sélection de trajectoire virtuel (1211) configuré comme un réseau de guides multi-lumières virtuel et aligné sur une image (1500) de l'ensemble de guidage de trajectoire (100), dans lequel ledit réseau de guides multi-lumière virtuel comprend une pluralité de canaux virtuels radialement et/ou circonférentiellement espacés autour d'un canal central virtuel, et dans lequel le canal central virtuel est aligné avec le centre de l'orifice ouvert (132p) de la plateforme (132).

3. Système selon la revendication 1, dans lequel la plateforme (132) comprend des indicateurs d'orientation visuelle (132i) sur une surface supérieure (132u) qui incluent un indicateur directionnel droit du patient (D_{R}), un indicateur directionnel gauche du patient (D_{L}) et un indicateur directionnel avant (D_{F}), dans lequel l'indicateur directionnel droit du patient (D_{R}), l'indicateur directionnel gauche du patient (D_{L}) et l'indicateur directionnel avant (D_{F}) sont fournis comme des marquages respectifs sur la surface supérieure (132u).

4. Système selon la revendication 1, dans lequel l'élément de guide de sélection de trajectoire (111,211) comprend une surface supérieure avec des indicateurs d'orientation visuelle (211i) incluant un indicateur directionnel droit du patient (D_{R}), un indicateur directionnel gauche du patient (D_{L}) et un indicateur directionnel avant (D_{F}).

5. Système selon la revendication 1, dans lequel l'élément de guide de sélection de trajectoire (111, 211) comprend un capuchon (211c) fixé de manière étanche à un corps principal (211b) et l'enveloppant, dans lequel le capuchon (211c) est situé au-dessus d'un réservoir de liquide (211fₜ), et dans lequel le réservoir de liquide (211fₜ) présente une largeur supérieure à la largeur de l'au moins une lumière remplie de fluide s'étendant longitudinalement et se fond dans l'au moins un canal rempli de fluide s'étendant longitudinalement.

6. Système selon la revendication 1, dans lequel la plateforme (132) est rectangulaire, dans lequel le système comprend en outre un élément de support tubulaire (204) maintenu par la plateforme (132) et s'étendant sous l'orifice ouvert (132p), et dans lequel l'orifice ouvert (132p) de la plateforme (132) comprend un périmètre avec un élément d'alignement (139) qui s'étend circonférentiellement autour d'un sous-ensemble du périmètre et qui reçoit de manière coulissante un élément d'alignement compatible (213) sur le guide du dispositif (311, 1311).

7. Système selon la revendication 1, dans lequel le guide de dispositif (311, 1311) est un premier guide de dispositif, et dans lequel le système comprend en outre un deuxième guide de dispositif qui est également prolongé de manière amovible et interchangeable à travers l'orifice de la plateforme et est directement fixé à la plateforme, dans lequel le deuxième guide de dispositif est
un guide central (1311c) avec un canal s'étendant longitudinalement (1312) qui est centré avec une ligne centrale s'étendant axialement du guide central (1311c).

8. Système selon la revendication 1, dans lequel l'élément de guidage de sélection de trajectoire (111, 211) est un réseau de guidage multi-lumière (211) comprenant une pluralité de lumières remplies de fluide (211f) espacées radialement et/ou circonférentiellement autour d'une lumière centrale remplie de liquide.

9. Système selon la revendication 1, dans lequel l'élément de guidage de sélection de trajectoire (111, 211) est un réseau de guidage multi-lumière (211) comportant une pluralité de lumières remplies de fluide (211f) radialement et/ou circonférentiellement espacées, et dans lequel les canaux remplis de fluide (211f) se terminent à une extrémité supérieure sous un capuchon (211c), et dans lequel le guide du dispositif (311, 1311) présente une extrémité supérieure qui est à la même hauteur que l'extrémité supérieure des canaux remplis de fluide.

10. Système selon la revendication 1, dans lequel l'ensemble de guidage de trajectoire (100) comprend en outre une paire de bras arqués latéralement espacés (101) qui maintiennent la plateforme (132) entre et au-dessus de la base (110) et seulement deux actionneurs (140a, 140b) pour le tangage et le roulis, et dans lequel l'ensemble de guidage de trajectoire (100) est dépourvu d'actionneurs de direction x-y.

11. Système selon la revendication 10, dans lequel la plateforme (132) est supportée de manière coulissante par les bras (101) pour permettre ainsi à la plateforme (132) de se déplacer de manière coulissante vers l'avant et vers l'arrière sur un chemin curviligne défini par les bras (101).

12. Système selon la revendication 9, dans lequel la pluralité de canaux remplis de fluide (211f) présentent une longueur commune.

13. Système selon la revendication 1, dans lequel l'élément de guidage de sélection de trajectoire (111, 211) est un réseau de guidage multi-lumière (211) comportant une pluralité de lumières remplies de fluide (211f) radialement et/ou circonférentiellement espacées, espacées autour d'une lumière centrale remplie de fluide, et dans lequel la pluralité de canaux remplis de fluide du réseau de guidage multi-lumière est au nombre de sept.

14. Système selon la revendication 1, dans lequel l'élément de guidage de sélection de trajectoire (111, 211) est un réseau de guidage multi-lumières (211) avec une pluralité de lumières remplies de fluide s'étendant longitudinalement et espacées (211f), dans lequel la pluralité de canaux remplis de fluide s'étendant longitudinalement comprend un canal central avec des canaux adjacents espacés autour du canal central, dans lequel le réseau de guidage multi-lumières (211) comprend des indicateurs d'orientation (211i) correspondant aux directions du patient à droite, à gauche et vers l'avant, et dans lequel la plateforme (132) présente des indicateurs d'orientation correspondants (132i).

15. Système selon la revendication 1, dans lequel la plateforme (132) comprend une surface supérieure (132u) avec des indicateurs d'orientation (132i) et une pluralité de lignes droites s'étendant radialement vers l'extérieur (135) sur la surface supérieure de celle-ci qui sont espacées circonférentiellement et s'étendent radialement à partir de l'orifice ouvert (132p).
